# EUROPEAN PATENT APPLICATION

(11) **EP 4 173 637 A1**
(43) Date of publication of application: **03.05.2023**
(21) Application number: 21832420.0
(22) Date of filing: 25.06.2021
(51) Int. Cl.: A61K 39/395, A61K 9/08, A61K 47/26, A61K 47/22, A61K 47/12, A61K 47/20, A61K 9/00, A61P 37/00, C07K 16/00, A61K 39/00

(54) **FORMULATION FOR ANTI-FCRN ANTIBODY**

(30) Priority: 29.06.2020 KR 20200079135
(71) Applicant: Hanall Biopharma Co., Ltd., Daejeon 34344 (KR)
(72) Inventor: AHN, Hyea Kyung, Yongin-si Gyeonggi-do 16938 (KR); KIM, Young Ju, Yongin-si Gyeonggi-do 17002 (KR); JUNG, Mi Jin, Suwon-si Gyeonggi-do 16493 (KR)
(74) Representative: Cooley (UK) LLP
(86) International application number: PCT/KR2021/008000
(87) International publication number: WO 2022/005113

(57) **Abstract**

In one aspect of the present invention, there is provided a pharmaceutical formulation having a pH of 4.0 to 8.0, comprising (a) an HL161BKN antibody or a fragment thereof, (b) at least one additive selected from mannitol, sorbitol, arginine, histidine, glycine and salts thereof, (c) a buffer system selected from citrate or histidine, and (d) a surfactant. HL161BKN present in the formulation has improved stability and is non-toxic, and thus has a high potential for industrial application.

## Description

### Technical Field

The present invention relates to a formulation optimized for HL161BKN, which is an anti-FcRn antibody.

### Background Art

The causes of autoimmune diseases have been studied for a long time from genetic, environmental and immunological perspectives, but the exact cause of the disease is still unknown. Many recent studies have revealed that a number of autoimmune diseases are caused by IgG-type autoantibodies. In fact, in studies for the diagnosis and treatment of autoimmune diseases, the relationship between the presence or absence of disease-specific autoantibodies and the therapeutic effect according to the reduction thereof has been widely investigated.

As a therapeutic agent for such autoimmune diseases, the first-line agent is a systemic high-dose steroid injection, and when symptoms are serious or difficult to control with steroids, high-dose IVIG (intravenous immunoglobulin) administration or plasmapheresis is applied. High-dose steroid may have a weak effect or have serious side effects when repeatedly used. In the case of IVIG and plasmapheresis, the cost of treatment is high and there are various side effects and risks of infection, so the development of a therapeutic agent in this treatment field is urgently needed.

On the other hand, recently, a therapeutic agent for autoimmune disease using an FcRn antibody is being studied (Korean Patent Publication No. 10-2014-0147606). It is a drug with a new strategy, in which the antibody blocks FcRn (Neonatal Fc Receptor), which is involved in the recycling of IgG, and increases the catabolism of IgG in the body, thereby lowering the levels of autoantibodies to treat the diseases. This anti-FcRn antibody is expected as a product that can solve the problems of existing therapeutic agents.

However, in order to apply this antibody to severe autoimmune diseases such as pemphigus vulgaris, neuromyelitis optica, and myasthenia gravis, which are caused by the generation of an autoantibody against an autoantigen in the body, a formulation optimized for this antibody and a formulation optimized for the administration method are required.

### Prior Art Document

### Patent Document

(Patent Document 1) KR 10-2014-0147606 A

### Disclosure of Invention

### Technical Problem

As a result of research in order to develop a formulation optimized for an anti-FcRn antibody for treating severe autoimmune diseases, the present inventors developed a buffer and formulation optimized for HL161BKN, which is an anti-FcRn antibody.

### Solution to Problem

In order to achieve the above object, in one aspect of the present invention, there is provided a pharmaceutical formulation comprising (a) an anti-FcRn antibody or a fragment thereof, (b) at least one additive selected from mannitol, sorbitol, arginine, histidine, glycine and salts thereof, (c) a buffer system selected from citrate or histidine, and (d) a surfactant.

### Effects of Invention

A pharmaceutical formulation according to the present invention having a pH of 4.0 to 8.0, comprising (a) an anti-FcRn antibody or a fragment thereof, (b) at least one additive selected from mannitol, sorbitol, arginine, histidine, glycine and salts thereof, (c) a buffer system selected from citrate or histidine, and (d) a surfactant, is a pharmaceutical composition optimized for HL161BKN, and it was confirmed that the stability of HL161BKN in the formulation was improved.

### Brief Description of Drawings

Figure 1 illustrates a result obtained by analyzing the thermal stability of HL161BKN using DSC (Differential Scanning Calorimetry).
Figure 2 illustrates a result obtained by confirming the 4-week accelerated stability of HL161BKN under conditions of sodium citrate-phosphate buffer at pH 5.0, 6.0, 7.0, and 8.0.
Figure 3 is a schematic diagram of the process for studying the formulation of HL161BKN.
Figure 4 illustrates a result obtained by confirming the amount of change in aggregates and fragments of HL161BKN (210 mg/mL) produced according to the various excipients as a result of excipient test.
Figure 5 illustrates a result obtained by analyzing the pattern of charge variants of HL161BKN (210 mg/mL) according to each excipient condition using CEX-HPLC.
Figure 6 illustrates a result of analysis of variance (ANOVA) of the amount of change in aggregates of HL161BKN according to the combination of excipients.
Figure 7 illustrates a result obtained by confirming the amount of change in aggregates and fragments of HL161BKN (210 mg/mL) produced according to the combination of excipients.
Figure 8 illustrates a result obtained by analyzing the amount of change in aggregates and fragments of HL161BKN according to the combination of excipients using Design of Experiment (DOE).
Figure 9 illustrates a result obtained by analyzing the pattern of charge variants of HL161BKN according to the combination of excipients using CEX-HPLC.
Figure 10 illustrates a result obtained by confirming the amount of change in aggregates and fragments of HL161BKN (210 mg/mL) in the additional excipient test.
Figure 11 illustrates a result obtained by analyzing the pattern of charge variants of HL161BKN according to the excipient conditions in the additional excipient test using CEX-HPLC.
Figure 12 illustrates a result obtained by confirming the effect of HL161BKN (210 mg/mL) on agitation stress according to the presence or absence of PSB20.
Figure 13 illustrates a result obtained by confirming the change in the monomer of HL161BKN according to the presence or absence of PSB20 in the agitation test.
Figure 14 illustrates a result obtained by confirming the amount of change in aggregates and fragments of HL161BKN (210 mg/mL) in the viscosity reducing excipient test.
Figure 15 illustrates a result obtained by confirming the change in aggregates and fragments of HL161BKN (210 mg/mL) in the first excipient screening.
Figure 16 illustrates a result obtained by analyzing the pattern of charge variants of HL161BKN according to the first excipient screening conditions using CEX-HPLC.
Figure 17 illustrates a result obtained by confirming the change in aggregates and fragments of HL161BKN (210 mg/mL) in the second excipient screening.
Figure 18 illustrates a result obtained by analyzing the pattern of charge variants of HL161BKN according to the second excipient conditions using CEX-HPLC.
Figure 19 illustrates a result obtained by confirming the change in the number of insoluble sub-visible particles of HL161BKN.
Figure 20 illustrates a result obtained by confirming the viscosity according to the concentration of HL161BKN in the HL161BKN formulation. Here, when HL161BKN was at a concentration of 170 mg/mL, the viscosity was confirmed to be 10 cP.

### Detailed Description for Carrying out the Invention

In one aspect of the present invention, there is provided a pharmaceutical formulation having a pH of 4.0 to 8.0, comprising (a) an anti-FcRn antibody or a fragment thereof, (b) at least one additive selected from mannitol, sorbitol, arginine, histidine, glycine and salts thereof, (c) a buffer system selected from citrate or histidine, and (d) a surfactant.

In addition, the pharmaceutical formulation may further comprise methionine. In addition, the pharmaceutical formulation may further comprise saccharides such as sucrose and trehalose.

The buffer system refers to a buffer that is resistant to changes in pH due to its acid-base counterpart.

As used herein, the term "histidine buffer" refers to a buffer containing a histidine ion. Here, specific embodiments of the histidine buffer may be any one selected from the group consisting of a histidine chloride buffer, a histidine acetate buffer, a histidine phosphate buffer, and a histidine sulfate buffer, but are not limited thereto.

As used herein, the term "citrate buffer" refers to a buffer containing a citrate ion. Here, specific embodiments of the citrate buffer may be any one selected from the group consisting of a sodium citrate buffer, a potassium citrate buffer, a calcium citrate buffer, and a magnesium citrate buffer, but are not limited thereto.

As used herein, the term "surfactant" refers to a pharmaceutically acceptable excipient that is used to protect a protein formulation against mechanical stress such as agitation and shearing. Specific embodiments of the surfactant may be any one surfactant selected from the group consisting of polysorbate 20, polysorbate 40, polysorbate 60, polysorbate 80, poloxamer, Triton, sodium dodecyl sulfate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauryl amidopropyl-betaine, cocamidopropyl-betaine, linoleamidopropyl-betaine, myristamidopropyl-betaine, palmitoylamidopropyl-betaine, isostearamidopropyl-betaine, myristamidopropyl-dimethylamine, palmitoylamidopropyl-dimethylamine, isostearamidopropyl-dimethylamine, sodium methyl cocacyl, sodium methyl oleyl-taurate, polyethylene glycol, polypropylene glycol, and copolymer of ethylene and propylene glycol. Here, the surfactant may be preferably polysorbate 20.

In addition, the pharmaceutical formulation may be an aqueous formulation, preferably an injectable liquid formulation.

Here, the anti-FcRn antibody may be HL161BKN.

The HL161BKN comprises a heavy chain variable region comprising H-CDR1 having the amino acid of SEQ ID NO: 5, H-CDR2 having the amino acid of SEQ ID NO: 6, and H-CDR3 having the amino acid of SEQ ID NO: 7, and a light chain variable region comprising L-CDR1 having the amino acid of SEQ ID NO: 8, L-CDR2 having the amino acid of SEQ ID NO: 9, and L-CDR3 having the amino acid of SEQ ID NO: 10.

In addition, the HL161BKN may comprise the heavy chain and light chain regions shown in Table 1 below. In addition, the heavy chain and light chain may be encoded by the nucleic acids shown in Table 2.

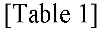

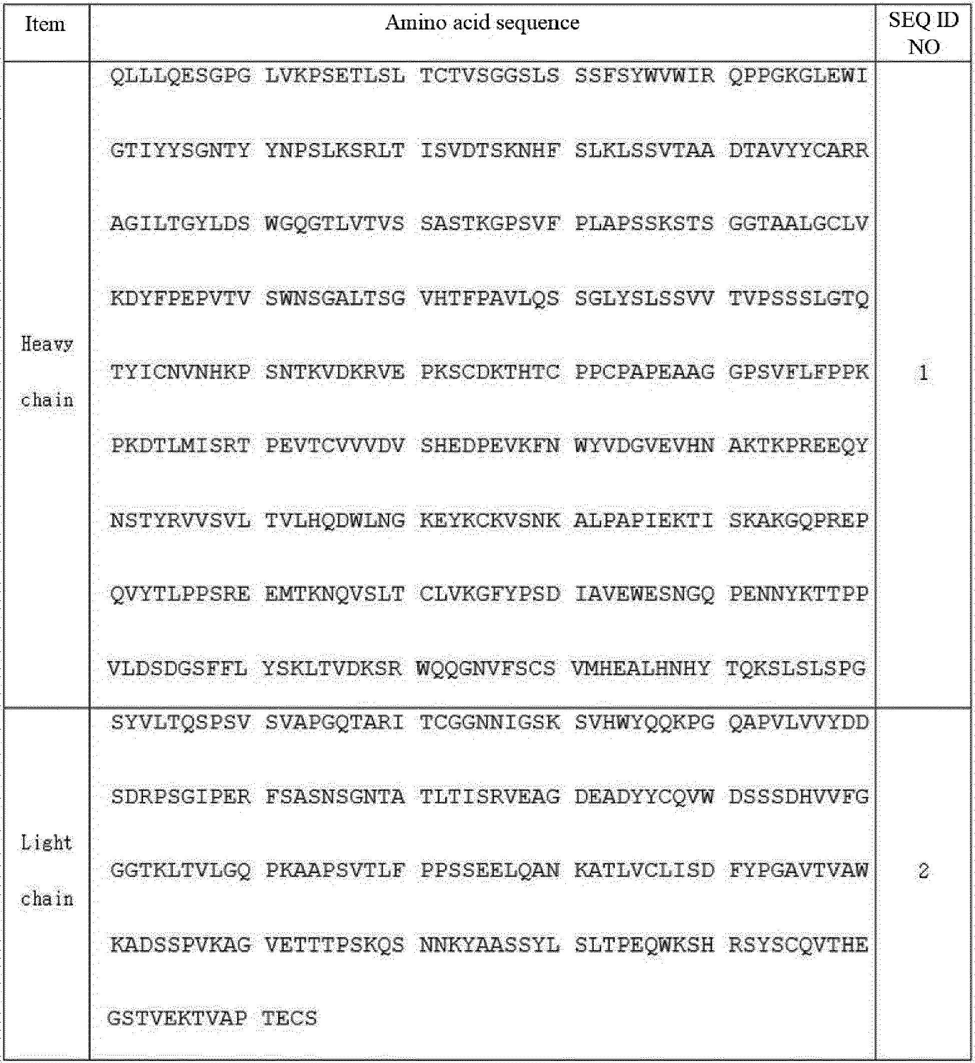

The glycosylation sites of the antibody are as follows: Asn301, N-glycan (G0F, G1F, G0-GlcNac, Man5).

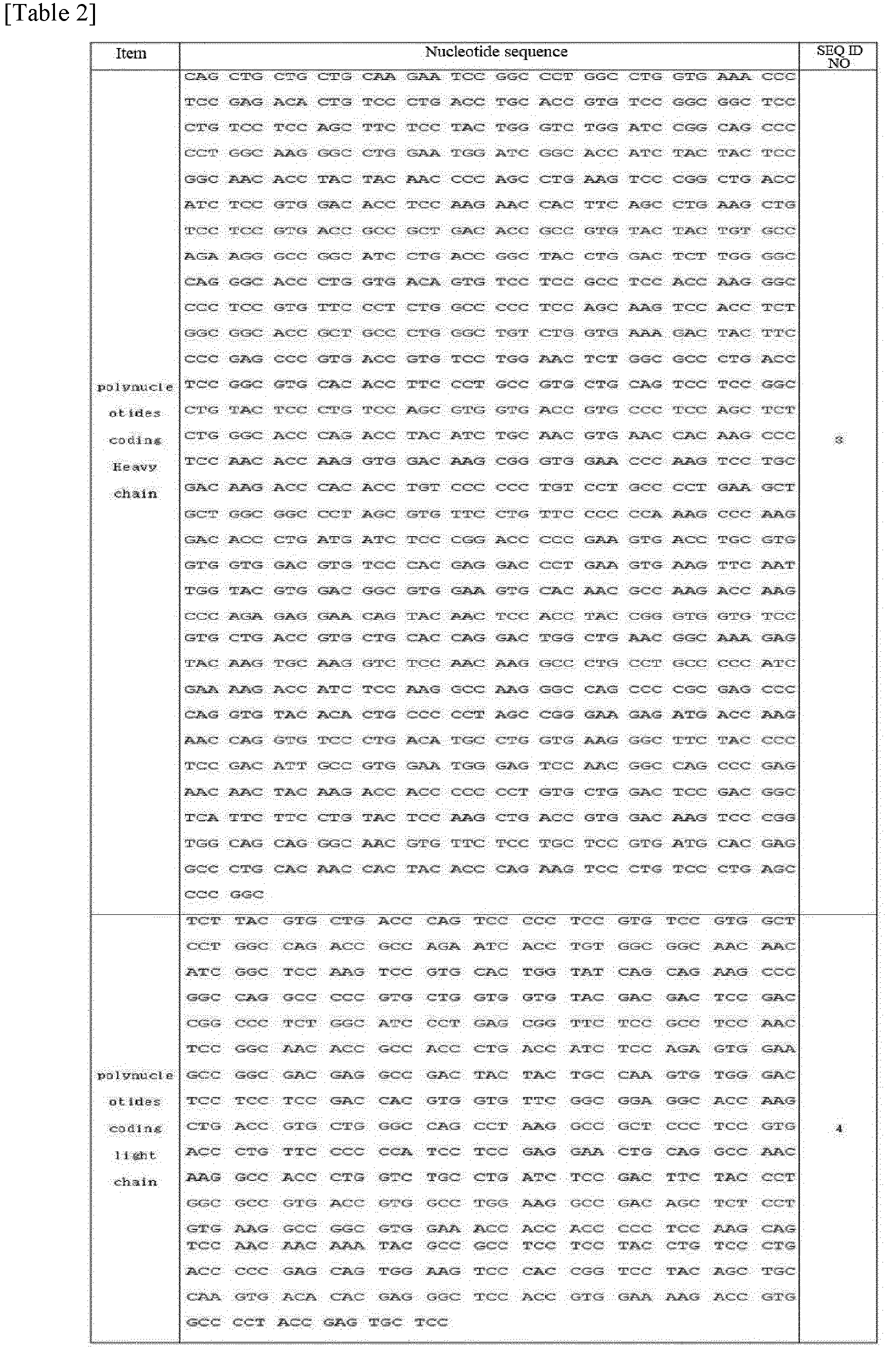

Here, the pharmaceutical formulation may have a viscosity of 20 cP or less. Specifically, the pharmaceutical formulation may have a viscosity of 1 cP to 20 cP. In addition, the pharmaceutical formulation may have a viscosity of 10 cP to 20 cP, and may have a viscosity of about 10 cP, about 11 cP, about 12 cP, about 13 cP, about 14 cP, about 15 cP, about 16 cP, about 17 cP, about 18 cP, about 19 cP, or about 20 cP.

In addition, the pharmaceutical formulation may have an osmolality of 250 mOs/kg to 500 mOs/kg. Specifically, the pharmaceutical formulation may have an osmolality of 300 mOs/kg to 450 mOs/kg or 350 mOs/kg to 400 mOs/kg. In addition, the pharmaceutical formulation may have an osmolality of about 250 mOs/kg, about 260 mOs/kg, about 270 mOs/kg, about 280 mOs/kg, about 290 mOs/kg, about 300 mOs/kg, about 310 mOs/kg, about 320 mOs/kg, about 330 mOs/kg, about 340 mOs/kg, about 350 mOs/kg, about 360 mOs/kg, about 370 mOs/kg, about 380 mOs/kg, about 390 mOs/kg, about 400 mOs/kg, about 410 mOs/kg, about 420 mOs/kg, about 430 mOs/kg, about 440 mOs/kg, about 450 mOs/kg, about 460 mOs/kg, about 470 mOs/kg, about 480 mOs/kg, about 490 mOs/kg, or about 500 mOs/kg.

In addition, the pharmaceutical formulation may include HL161BKN at a concentration of 50 mg/mL to 250 mg/mL. Specifically, the pharmaceutical formulation may include HL161BKN at a concentration of 60 mg/mL to 250 mg/mL, 70 mg/mL to 250 mg/mL, 80 mg/mL to 250 mg/mL, 90 mg/mL to 250 mg/mL, or 100 mg/mL to 250 mg/mL. In addition, the pharmaceutical formulation may include HL161BKN at a concentration of 120 mg/mL to 230 mg/mL, 150 mg/mL to 220 mg/mL, or 180 mg/mL to 210 mg/mL. In addition, the pharmaceutical formulation may include HL161BKN at a concentration of about 50 mg/mL, about 60 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, about 200 mg/mL, about 210 mg/mL, about 220 mg/mL, about 230 mg/mL, about 240 mg/mL, or about 250 mg/mL.

In addition, the pharmaceutical formulation may have a pH of 4.0 to 8.0. Specifically, the pharmaceutical formulation may have a pH of 4.0 to 7.0. Preferably, the pharmaceutical formulation may have a pH of 5.0 to 6.0. In addition, the pharmaceutical formulation may have about pH 5.0, about pH 5.1, about pH 5.2, about pH 5.3, about pH 5.4, about pH 5.5, about pH 5.6, about pH 5.7, about pH 5.8, about pH 5.9, about pH 6.0, about pH 6.1, about pH 6.2, about pH 6.3, about pH 6.4, about pH 6.5, about pH 6.6, about pH 6.7, about pH 6.8, about pH 6.9, or about pH 7.0.

In addition, the additive may be included at a concentration of 10 mM to 400 mM. Here, as the additive, mannitol, sorbitol, arginine, histidine or glycine may be used alone, and two or more may be used in combination. Specifically, the additive may be included at a concentration of 10 mM to 400 mM, 20 mM to 300 mM, 50 mM to 250 mM, or 100 mM to 150 mM, respectively. Specifically, the additive may be included at a concentration of about 10 mM, about 20 mM, about 30 mM, about 40 mM, about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 180 mM, about 190 mM, about 200 mM, about 210 mM, about 220 mM, about 230 mM, about 240 mM, about 250 mM, about 260 mM, about 270 mM, about 280 mM, about 290 mM, or about 300 mM, respectively.

In addition, two of the additives may be used in combination. In one embodiment, the additive may include mannitol and sorbitol. In one embodiment, the additive may include mannitol and arginine. In one embodiment, the additive may include mannitol and histidine. In one embodiment, the additive may include mannitol and glycine. In one embodiment, the additive may include sorbitol and arginine. In one embodiment, the additive may include sorbitol and histidine. In one embodiment, the additive may include sorbitol and glycine. In one embodiment, the additive may include arginine and histidine. In one embodiment, the additive may include arginine and glycine. In one embodiment, the additive may include histidine and glycine. Here, each additive may be included in a pharmaceutical formulation at the above-mentioned concentration.

One embodiment of the pharmaceutical formulation may be a pharmaceutical formulation having a pH of 5.0 to 6.0, comprising (a) 100 mg/mL to 250 mg/mL of an anti-FcRn antibody, (b) 50 to 250 mM L-arginine or a hydrochloride salt thereof, (c) 50 to 250 mM L-histidine, and (d) 0.01 to 0.05% polysorbate 20.

Here, the anti-FcRn antibody is as described above. In addition, the pharmaceutical formulation may be an aqueous formulation, and may be an injectable liquid formulation. In addition, the above-mentioned pharmaceutical formulation may be administered subcutaneously.

In addition, it was confirmed that the pharmaceutical formulation is very stable under accelerated conditions. Specifically, the content of aggregates and fragments may be about 10% or less as a result of a 6-month test under an accelerated condition (25°C, a relative humidity of 60%). In addition, the content of aggregates and fragments under the above condition may be about 9% or less, about 8% or less, about 7% or less, about 6% or less, about 5.5% or less, or about 5.0% or less.

In addition, it was confirmed that the pharmaceutical formulation is very stable even under a long-term storage condition. Specifically, the content of aggregates and fragments under the condition of 5°C and 36 months may be about 10% or less. In addition, the content of aggregates and fragments under the above condition may be about 9% or less, about 8% or less, about 7% or less, about 6% or less, about 5% or less, about 4% or less, about 3% or less, about 2% or less, about 1.8% or less, about 1.5% or less, or about 1.2% or less.

In addition, the pharmaceutical formulation may be used for the treatment of autoimmune diseases. Here, the autoimmune disease may be any one selected from the group consisting of myasthenia gravis (MG), thyroid eye disease (TED), warm autoimmune hemolytic anemia (WAIHA), neuromyelitis optica (NMO), immune thrombocytopenic purpura (ITP), pemphigus vulgaris (PV), chronic inflammatory demyelinating polyneuropathy (CIDP), lupus nephritis (LN), and membranous nephropathy (MN).

### Screening of formulation optimized for HL161BKN

HL161BKN was prepared at a concentration of about 210 mg/mL for each condition and stored for 4 weeks under accelerated conditions of 40°C, and the analysis of concentration (A280), turbidity (A340), purity (SEC-HPLC, CEX-HPLC), viscosity, osmolality, insoluble sub-visible particle (MFI) and the like was performed to evaluate the stability of the sample and suitability for subcutaneous administration.

First, a screening test was performed on 11 excipients that are frequently used in existing antibody products. As a result, it was confirmed that L-histidine, L-arginine hydrochloride, L-glycine, D-sorbitol, and D-mannitol had the effect of reducing the formation of aggregates.

A synergistic effect was tested through a combination of five selected excipients. As a result, it was confirmed that L-arginine hydrochloride reduced the formation of aggregates at a statistically significant level (p<0.01).

By combining the results of comparison of effect values on aggregates and fragmentation, L-histidine and D-mannitol were selected as excipients. In addition, as a result of screening for additional excipients, L-methionine, which exhibited the effect of reducing the formation of aggregates, was additionally selected. In addition, it was confirmed that histidine can be used as a basal buffer. In addition, high-purity 0.02% polysorbate 20, which exhibited the effect of inhibiting the formation of aggregates due to agitation stress that may occur during storage and transport of the product, was selected.

Next, the first concentration screening test of the selected histidine basal buffer and L-arginine hydrochloride, D-mannitol, and L-methionine excipients was performed. Here, the concentration of PBS20 was fixed at 0.02% and performed. As a result, high stability was confirmed under conditions of 50 mM histidine basal buffer without excipients. In addition, the amount of aggregates and fragments increased according to the excipient conditions did not show a significant difference, but the formation of aggregates was reduced as the concentration of L-arginine hydrochloride added was increased.

On the other hand, it was confirmed that there was little difference according to the presence or absence of D-mannitol and the concentration of L-methionine. Therefore, L-arginine hydrochloride and 0.02% PSB20 were selected in a L-histidine basal buffer as a HL161BKN formulation.

Finally, in order to select the optimal concentrations of L-histidine and L-arginine hydrochloride, the second concentration screening was performed with two batches of HL161BKN. As a result, the amount of aggregates and fragments increased according to the concentration conditions of the excipient did not show a significant difference, and it was confirmed that the measured values of viscosity and osmolality under all conditions were 20 cP or less and 250 to 500 mOsmol/kg, which were suitable for subcutaneous administration.

In addition, as a result of performing the analysis of insoluble sub-visible particles using micro flow imaging (MFI) for each condition sample, the condition in which the increase in the number of insoluble sub-visible particles was the least was confirmed; and the stability and subcutaneous injection (SC) administration suitability through the purity (aggregates and fragments), viscosity, and osmolality tests were evaluated to select the final formulation.

In conclusion, about 100 mM L-histidine, about 100 mM L-arginine hydrochloride, and about 0.02% polysorbate 20 (pH 6.0) were selected as a formulation for non-clinical and phase 1 clinical trials of HL161BKN.

Hereinafter, the present invention will be described in more detail through the examples. These examples are only for illustrating the present invention, and it will be apparent to those of ordinary skill in the art that the scope of the present invention is not to be construed as being limited by these examples.

### I. Preparation of HL161BKN

### Prepartion Example 1. Prepation of gene construct and vector containing them for preparing HL161 antibody

In order to prepare HL161BKN, a polynucleotide having the nucleic acid sequence of SEQ ID NO: 3 encoding a heavy chain comprising the amino acid sequence of SEQ ID NO: 1 was loaded into the pCHO 1.0 vector (Life Technologies). In addition, a polynucleotide having the nucleic acid sequence of SEQ ID NO: 4 encoding a light chain comprising the amino acid sequence of SEQ ID NO: 2 was loaded into the pCHO 1.0 vector.

### Prepartion Example 2. Prepation of HL161BKN cell line and production of HL161BKN

CHO-S cells were transformed using the expression vector prepared in Prepartion Example 1, and subjected to the selection process of methotrexate and puromycin, and then a final production cell line was prepared. The prepared cell line was prepared and stored as a cell bank, and used for HL161BKN production. The antibody production was performed in a bioreactor containing a culture medium (Dynamis medium + 8 mM L-glutamine) by adding an additional medium (EFB+) every 2 days, and the supernatant was recovered after culturing for about 15 days. Thereafter, a Protein A column was performed, and viral inactivation was performed at low pH, and then anion exchange chromatography (AEX) and cation exchange chromatography (CEX) were performed. Thereafter, after concentration and buffer exchange (Ultrafiltration/Diafiltration), it was purified by sterile filtration. The quality of the produced antibody sample was confirmed through analysis such as SEC-HPLC, CE-SDS, cIEF, ELISA, potency, and concentration.

### Prepartion Example 3. Analysis of basic properties of HL161BKN

Thermal stability of HL161BKN, stability test under accelerated conditions according to pH, and solubility test were performed. For thermal stability, Tm values were analyzed by DSC (differential scanning calorimetry), and for accelerated condition experiment for each pH, the degree of the formation of aggregates and fragments was monitored while storing at 37°C for 1 month.

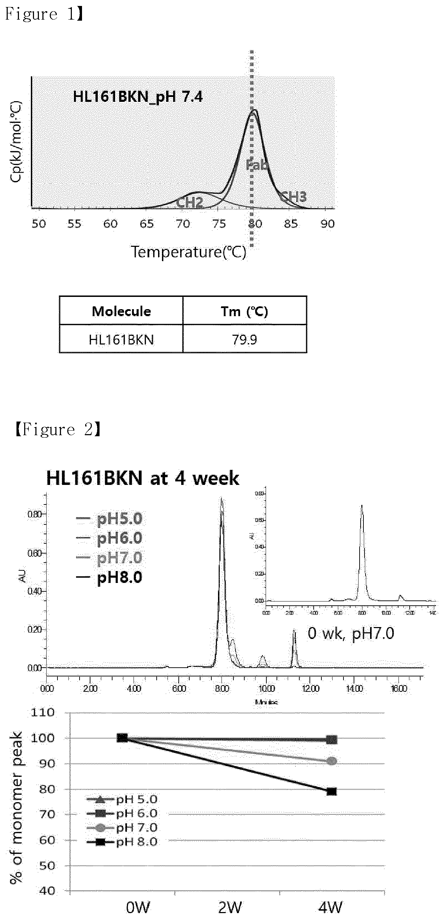

For the DSC for Tm value analysis, the equipment from Osong Hi-tech Medical Industry Promotion Foundation was used. Experiments were conducted in the range of 25°C to 100°C, and the results were confirmed as shown in Figure 1. A typical DSC histogram of the IgG1 type was shown, and curves according to the CH domain and Fv domain were confirmed. HL161BKN exhibited a high Tm of 79.9°C and was analyzed to be thermodynamically very stable.

In addition, using sodium citrate-phosphate buffers at pH 5.0, 6.0, 7.0, and 8.0, HL161BKN was prepared at a concentration of 10 mg/mL. Thereafter, while storing at 37°C for 4 weeks, the degree of the formation of aggregates or fragments was confirmed using SEC-HPLC. As a result, the formation of aggregates or fragments was increased as the pH was increased (Figure 2).

Based on this result, HL161BKN was prepared at a concentration of 100 mg/mL using buffers having a low concentration or high concentration, which is low pH of 5.0 and 6.0, and then the stability test was performed while storing for 4 weeks under accelerated conditions of 40°C. As a result, HL161BKN exhibited a tendency to be stable under low pH conditions, pH 5.0 to 6.0, and maintained a high level of monomer overall under all conditions.

In addition, in order to confirm the solubility of HL161BKN, it was concentrated in 6 steps to a concentration of 10 to 300 mg/mL, and samples were collected step by step and visually observed, and the analysis of A280 nm (concentration), A340 nm (turbidity), and SEC-HPLC purity was performed. As a result, it was confirmed that the turbidity of HL161BKN was gradually increased as the concentration thereof was increased, but there was little change in the monomer purity at a concentration of up to 268 mg/mL.

### II. Selection of formulation optimized for HL161BKN

### Prepartion Example 1. Screening of formulation optimized for HL161BKN

The purpose of this experiment is to select the formulation of the drug substance and drug product for the development of high concentration subcutaneous administration products of HL161BKN. In this experiment, three production batches of HL161BKN-B005, HL161BKN-B018, and HL161BKN-B021 were used, and the reagents and instruments used are as follows (Table 3).

**[Table 3]**

| **Components** | **Manufacturer** |
|---|---|
| D-(+)-Trehalose dihydrate | Sigma |
| D-Mannitol | Sigma |
| D-Mannitol | Avantor |
| D-Sorbitol | Sigma |
| L-Arginine monohydrochloride | Merck |
| L-Histidine monohydrochloride monohydrate | Merck |
| L-Histidine | Sigma |
| L-Histidine | Avantor |
| Polysorbate 20 (PSB20) | Sigma |
| Polysorbate 20 (PSB20) | Avantor |
| Polysorbate 80 (PSB80) | Merck |
| Sodium chloride | Merck |
| Sucrose | Bioshop |
| Sucrose | Avantor |
| L-Glycine | Merck |
| L-Methionine | Avantor |
| L-Lysine-monohydrochloride | Tianjin Tianyao pharmaceuticals |
| L-Valine | Evonik |
| Sodium sulfate anhydrus | Deajung |
| Ammonium chloride | Sigma |
| Citric acid, anhydrus | Avantor |
| Citric acid, anhydrus | Merck |
| Acetonitrile | B&J |
| Tri-sodium citrate, dihydrate | Merck |
| Tri-sodium citrate, dihydrate | Avantor |
| 1.5 mL microcentrifuge tube | Axygen |
| Amicon^{®} ultra centricon, 15 mL | Millipore |
| Amicon^{®} ultra centricon, 4 mL | Millipore |

The instruments used for this formulation test are as follows (Table 4).

**[Table 4]**

| **Instruments** | **Manufacturer** | **Catalog number** |
|---|---|---|
| Centrifuge | Gyrogen | 1580-MGR |
| mv-ROC Viscometer | Rheosense | mv-ROC |
| Osmometer | Gonatec | Osmomat 3000 |
| Temperature & Humidity Chamber | Jeio tech | TH-TG-300 |
| Nanodrop | Thermoscientific | Nanodrop2000 |
| Propac WCX-10 | Dionex | 054993 |
| TSK gel G3000SWXL | TOSOH | 5788 |
| TSK SWXL guard column | TOSOH | 8543 |
| Water bath | Daihan | DH.WB000111 |
| Waters alliance HPLC | Waters | E2695 / 2489 |
| MyLab intelli mixer | MyLab | SLRM-3 |
| MFI 5200 particle analyzer | ProteinSimple | 4000-015-001 |
| Stat-ease Design-Expert^{®} software | Stat-Ease | Version 7.0 |

### Prepartion Example 2. Test method

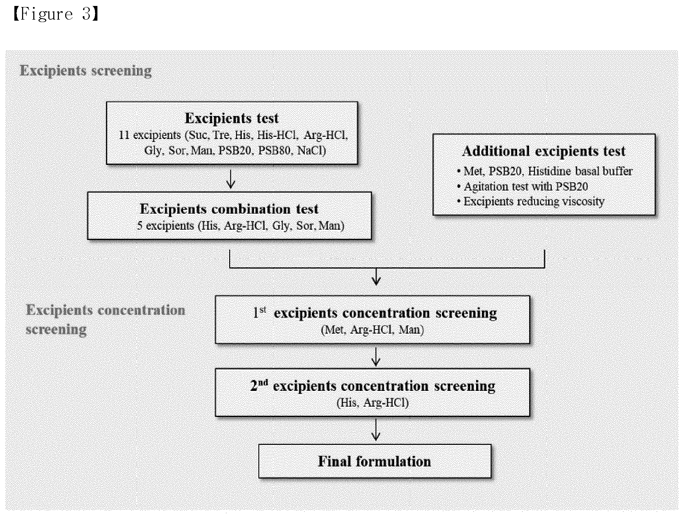

The formulation selection test for HL161BKN was largely divided into tests of an excipient screening and an excipient concentration screening, and performed. Specifically, an experiment was performed in the same manner as in Figure 3.

### Example 1. Excipient screening

### Example 1.1. Excipient test

Sucrose, D-trehalose, D-mannitol, D-sorbitol, L-arginine HCl, L-histidine HCl, L-histidine, L-glycine, polysorbate 20, polysorbate 80, and sodium chloride (NaCl), which were 11 excipients that are frequently used in antibody products currently on the market, were selected. Specifically, the excipient test was performed in 12 buffer conditions using 5 mM sodium citrate (pH 6.0) as a basal buffer (Table 5).

HL161BKN was concentrated to 1 mL or less using AMICON^{®} (Cutoff MW. 30,000), and then a buffer was exchanged with the corresponding buffer conditions to obtain a final concentration of 210 mg/mL. Samples for each condition were prepared by 0.3 to 0.5 mL, and stored in 1.5 mL microcentrifuge tubes at 40°C for 4 weeks. Samples were sampled at week 0, week 2, and week 4 to evaluate changes in concentration (A280), turbidity (A340), and purity (SEC-HPLC, CEX-HPLC).

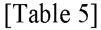

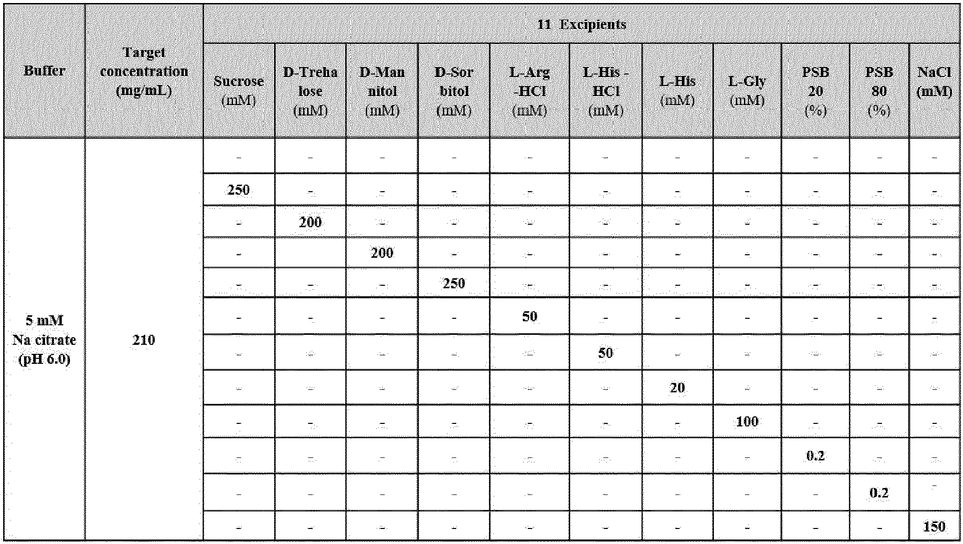

### Example 1.2. Excipient combination test

When combining the five excipients (L-histidine, L-arginine hydrochloride, L-glycine, D-sorbitol, and D-mannitol) selected in Example 1.1, it was confirmed whether there was a synergistic effect. Specifically, 17 condition tests were planned in a 2-level factorial (2ⁿ⁻¹) design using DOE (design of experiments) software (Stat-ease DESIGN-EXPERT^{®}, version 7.0) (Table 6). Specifically, a total of 12 condition tests were performed: 5 mM sodium citrate (pH 6.0) basal buffer condition and 11 excipient combination conditions. In addition, the test results of Example 1.1 were used for the five excipients only condition tests.

The test method was performed in the same manner as in Example 1.1, and the osmolality of the buffer and sample for each condition was further measured, and ANOVA analysis and effect values were calculated using the DOE Software.

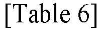

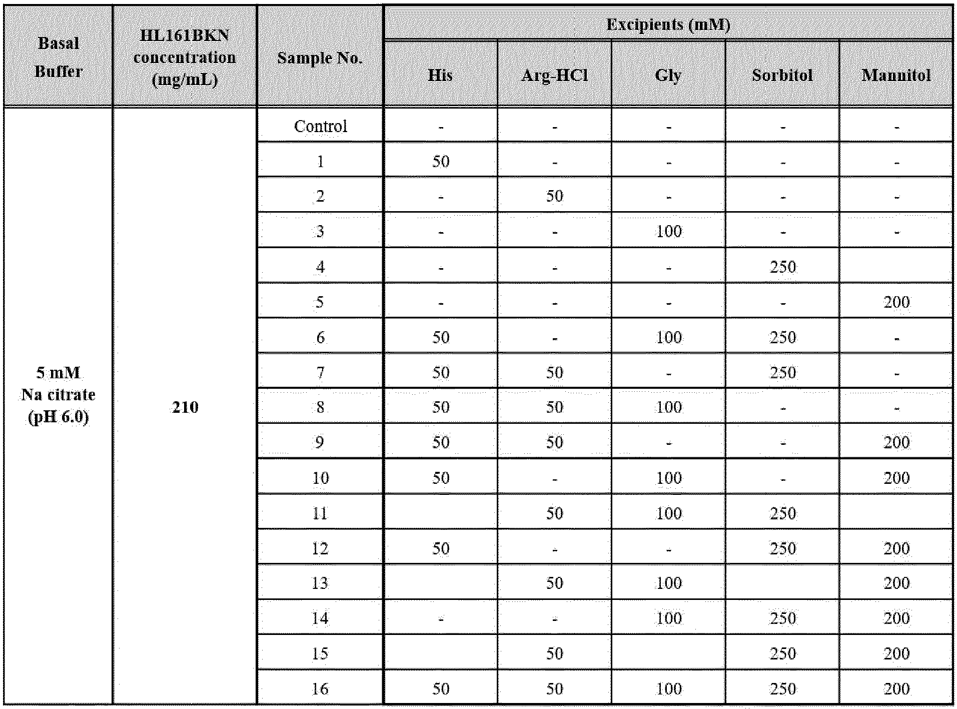

### Example 1.3. Test for additional excipient

An additional analysis of excipients for the HL161BKN formulation test was performed.

An additional test of L-methionine, which is known to have the effect of reducing covalent aggregates; and a test of changing the sodium citrate (pH 6.0) basal buffer, which may cause pain during injection, to histidine (pH 6.0) were performed. In addition, when low-quality PSB20 (Polysorbate 20) containing a lot of peroxide is used, the antibody stability is reduced due to oxidation, so it was changed to high-quality PSB20 for formulation. Thereafter, the effect on agitation stress was confirmed at a concentration of 0.02%, which is commonly used in the formulation. Then, an excipient capable of reducing the viscosity of the high concentration antibody product was screened.

### Example 1.3.1. Test for L-methionine and histidine basal buffer

In order to confirm the effect of L-methionine and whether the basal buffer, 5 mM sodium citrate (pH 6.0), can be changed to histidine (pH 6.0), a test was performed under the six conditions in Table 7. In the case of the histidine basal buffer, L-histidine and L-histidine hydrochloride were mixed to prepare them at a pH of 6.0 and used. The test method was performed in the same manner as in Example 1.1.

**[Table 7]**

| **HL161BKN concentration (mg/mL)** | **Conditions** | **Buffer** |
|---|---|---|
| **210** | **1** | **5 mM Na Citrate (pH 6.0)** |
| | **2** | **5 mM Na Citrate, 50 mM Methionine (pH 6.0)** |
| | **3** | **5 mM Na Citrate, 50 mM Histidine (pH 6.0)** |
| | **4** | **5 mM Na Citrate, 0.02% PSB20 (pH 6.0)** |
| | **5** | **10 mM Histidine (pH 6.0)** |
| | **6** | **50 mM Histidine (pH 6.0)** |

### Example 1.3.2. Polysorbate 20 (PSB20) test

In order to confirm the protective effect of PSB20 against agitation stress, a test was performed under the four conditions in Table 8. 0.5 mL of the respective samples prepared in the same manner as in Example 1.1 was placed in a 1.5 mL microcentrifuge tube, and equiped on a MyLab intelli mixer, and rotated at 10 rpm at room temperature for 1 week, and then concentration, turbidity, and purity (SEC-HPLC) analysis was performed.

**[Table 8]**

| **Target concentration (mg/mL)** | **Buffer** | **PSB20** | **Agitation** |
|---|---|---|---|
| **210** | **5 mM Na citrate (pH6.0)** | - | - |
| | | **-** | **+** |
| | | **0.02%** | - |
| | | **0.02%** | **+** |

### Example 1.3.3. Screening of excipient for viscosity reduction

In order to develop HL161BKN high concentration products for subcutaneous administration, the eight excipients, which are generally known to reduce viscosity, were tested. A total of 9 excipient screening tests were performed using 50 mM histidine (pH 6.0) as a basal buffer (Table 9).

Samples were prepared by 1 mL for each condition in the same manner as in Example 1.1, and the viscosity was measured at 25°C. In addition, changes in concentration (A280) and purity (SEC-HPLC) for each condition were evaluated while performing the 4-week accelerated stability test.

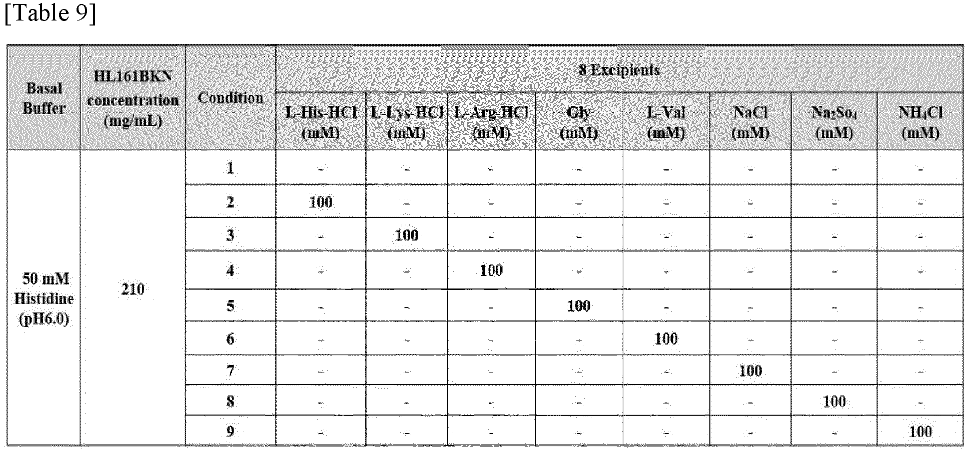

### Example 1.4. Excipient concentration screening

### Example 1.4.1. First excipient concentration screening

DOE test of 2 level factorial (2ⁿ) design was planned for the 50 mM histidine (pH 6.0) basal buffer selected through Examples 1.1, 1.2 and 1.3 and three excipients (L-methionine, L-arginine hydrochloride, and D-mannitol) of four excipients (L-methionine, L-arginine hydrochloride, D-mannitol, and PSB20), and excipient concentration screening was performed under a total of 9 conditions (Table 10).

The experiment was performed in the same manner as in Example 1.1, and changes in concentration (A280), turbidity (A340), purity (SEC-HPLC, CEX-HPLC), viscosity, and osmolality were analyzed.

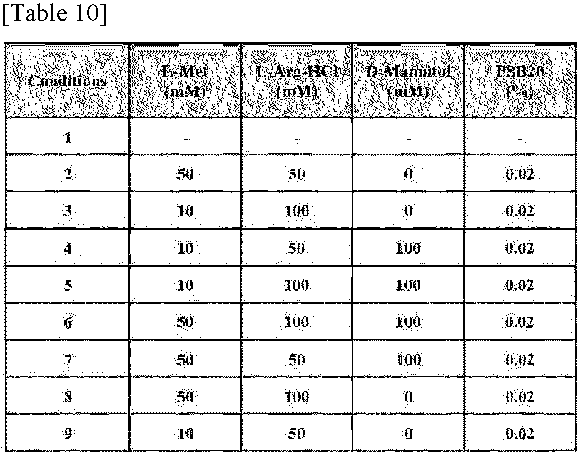

### Example 1.4.2. Second excipient concentration screening

In order to investigate the optimal concentration of L-arginine hydrochloride among the final selected histidine basal buffer and two excipients (L-arginine hydrochloride and PSB20), a 4-week 40°C accelerated stability test under four conditions was performed using two batches of HL161BKN B018 and B021 (Table 11). The test was performed in the same manner as in Example 1.1, and concentration (A280), turbidity (A340), purity (SEC-HPLC, CEX-HPLC), viscosity, osmolality, and insoluble sub-visible particle (Micro flow imaging; MFI) were analyzed.

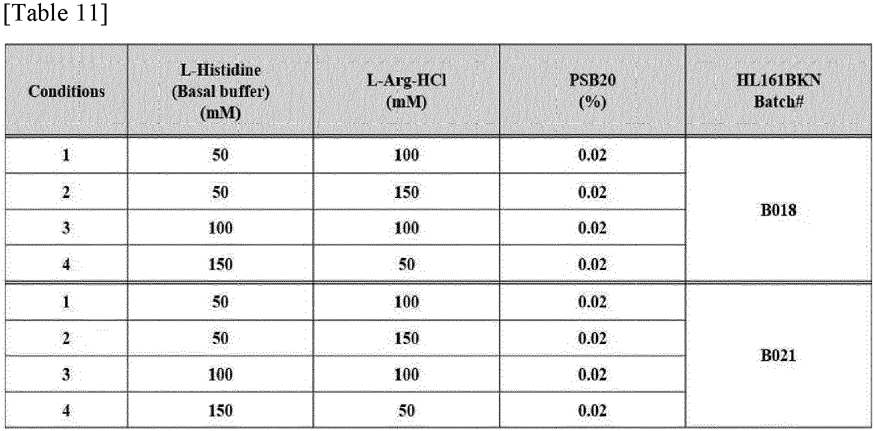

### Example 2. Results of Excipient screening test

### Example 2.1. Results of Excipient test

In order to evaluate the effect of 11 excipients selected for excipient screening on the stability of HL161BKN samples, analysis of concentration, turbidity, purity, aggregates, fragments, and charge variants was performed at week 0, week 2, and week 4 under accelerated conditions of 40°C. Through these analysis, 5 excipients (L-arginine hydrochloride, L-histidine, D-mannitol, L-glycine, and D-sorbitol), which had a good effect of inhibiting the formation of aggregates and fragments, were selected.

### Example 2.1.1. Results of concentration (A280) and turbidity (A340) analysis

HL161BKN concentration was increased for 4 weeks (Table 12), which was assumed to be due to buffer evaporation under accelerated conditions of 40°C.

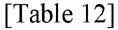

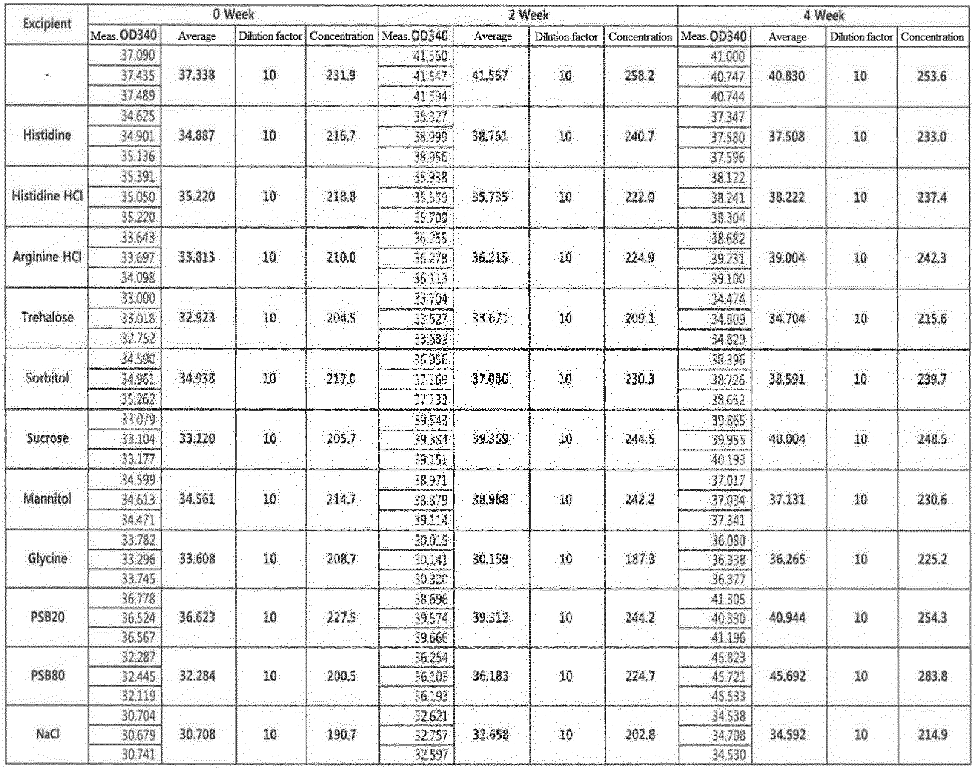

On the other hand, the turbidity was not increased in most conditions, or the amount of change was 0.020 or less. However, it was observed that it was visually cloudy in the PSB20 condition, and it was confirmed that the turbidity (A340) was also greatly increased (Table 13).

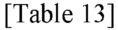

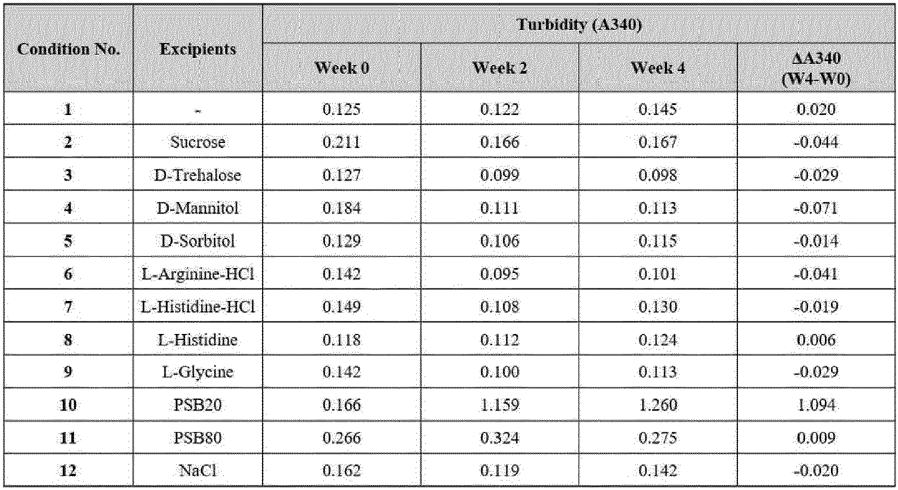

### Example 2.1.2. Results of aggregate and fragment analysis

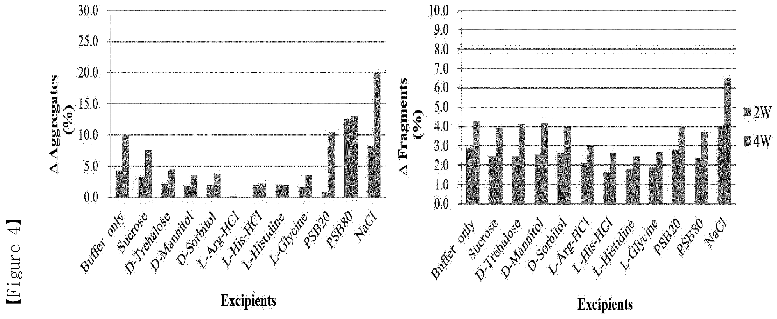

SEC-HPLC was used to compare and evaluate the amount of aggregates and fragments increased for each excipient condition (Table 14). Compared with the basal buffer condition, L-arginine hydrochloride, L-histidine, L-histidine hydrochloride, D-mannitol, L-glycine, and D-sorbitol effectively inhibited the formation of aggregates. In addition, 0.2% PSB20, 0.2% PSB80, and NaCl rather increased the formation of aggregates. In addition, L-histidine, L-histidine hydrochloride, L-arginine hydrochloride, and L-glycine were the excipients inhibiting the formation of fragments, and NaCl increased the formation of fragments (Table 14 and Figure 4).

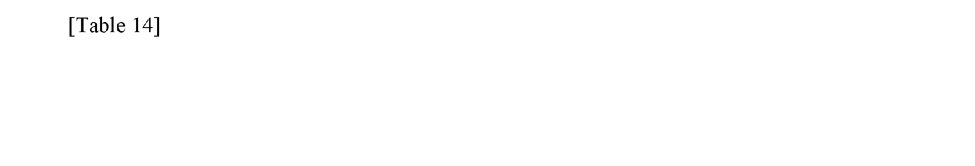

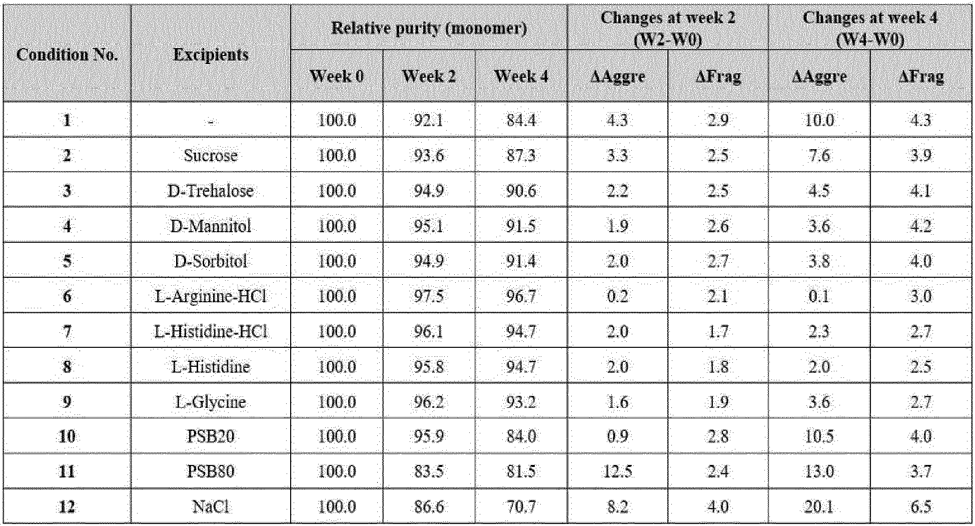

### Example 2.1.3. Results of charge variant analysis

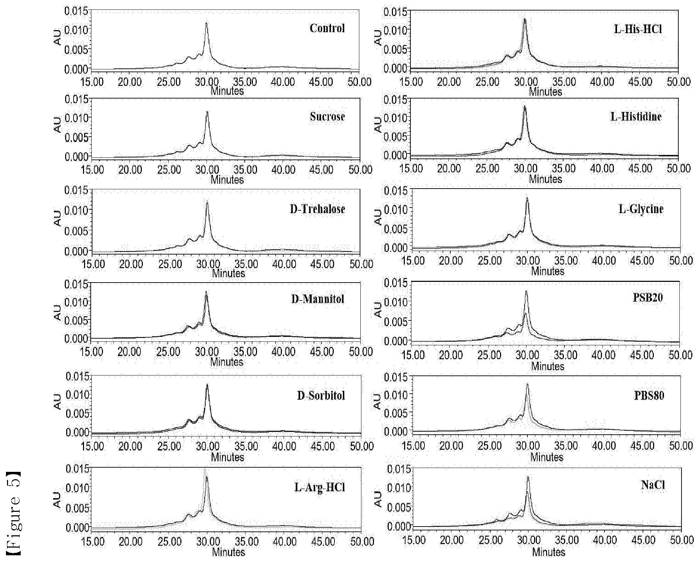

The pattern of changes in charge variants of HL161BKN according to each excipient condition was confirmed using CEX-HPLC. As a result, no distinct changes in charge variants could be observed. However, the main peak was greatly reduced in PSB20 and NaCl conditions (Figure 5). This was expected as a result of increased aggregates.

### Example 2.2. Results of excipient combination test

The effect of the combination of five excipients selected in the excipient test of Example 1.1 on stability of the HL161BKN sample was evaluated. Specifically, the 40°C 4-week accelerated test was performed, and L-arginine hydrochloride, L-histidine, and D-mannitol, which effectively inhibited the formation of aggregates and fragments, were selected.

In order to confirm whether the excipients selected through the excipient screening test have a synergistic effect through combination, a condition test was planned using design of experiments (DOE) software. A total of 12 condition tests were performed, including a basal buffer condition and 11 excipients combination conditions. The test results of Example 1.1 were used for the five excipients alone condition test (Table 15).

When the results of comparison of the amount of change in aggregates were analyzed by ANOVA, an excipient that reduced the formation of aggregates at a statistically significant level (p<0.01) was found. However, there were no excipients that reduced the formation of fragments at a statistically significant level.

On the other hand, in the case of HL161BKN, the formation pattern of aggregates was different depending on the combination of excipients, but there was no synergistic effect by the combination of excipients. The kinds of excipient was selected with reference to the ANOVA analysis and the comparison ranking of effect values.

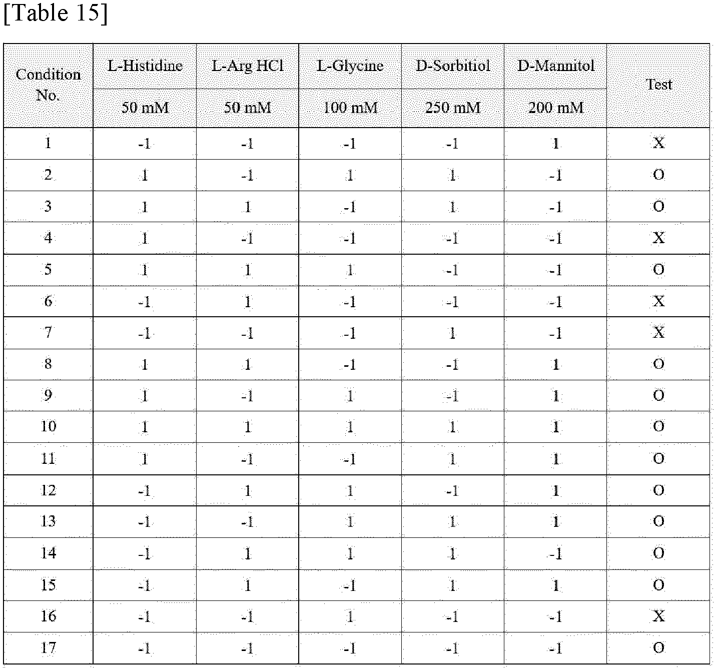

### Example 2.2.1. Results of concentration (A280) and turbidity (A340) analysis

The concentration of HL161BKN was increased for 4 weeks, which was assumed to be due to buffer evaporation under accelerated conditions of 40°C (Table 16). On the other hand, turbidity was not increased or showed a slight increase to 0.054 or less (Table 17).

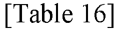

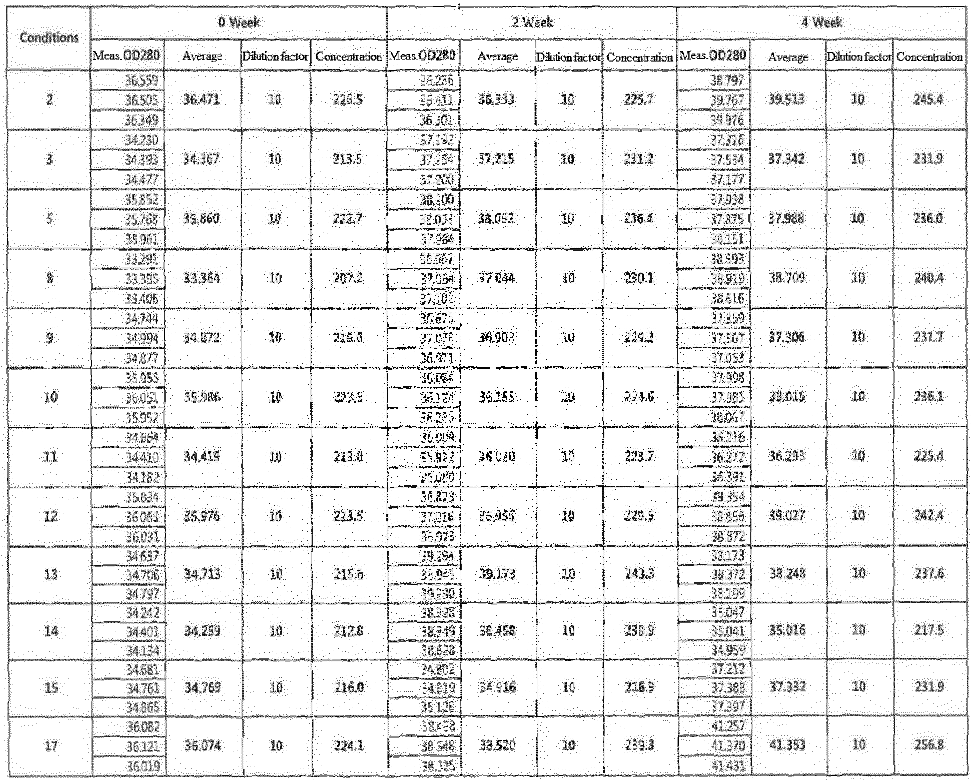

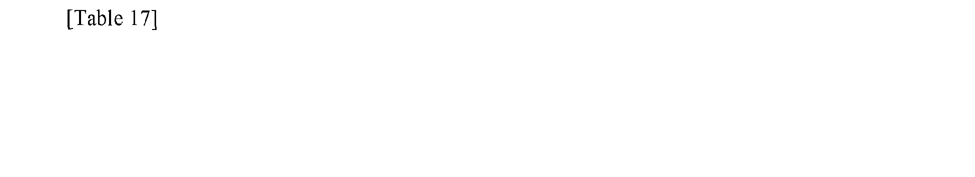

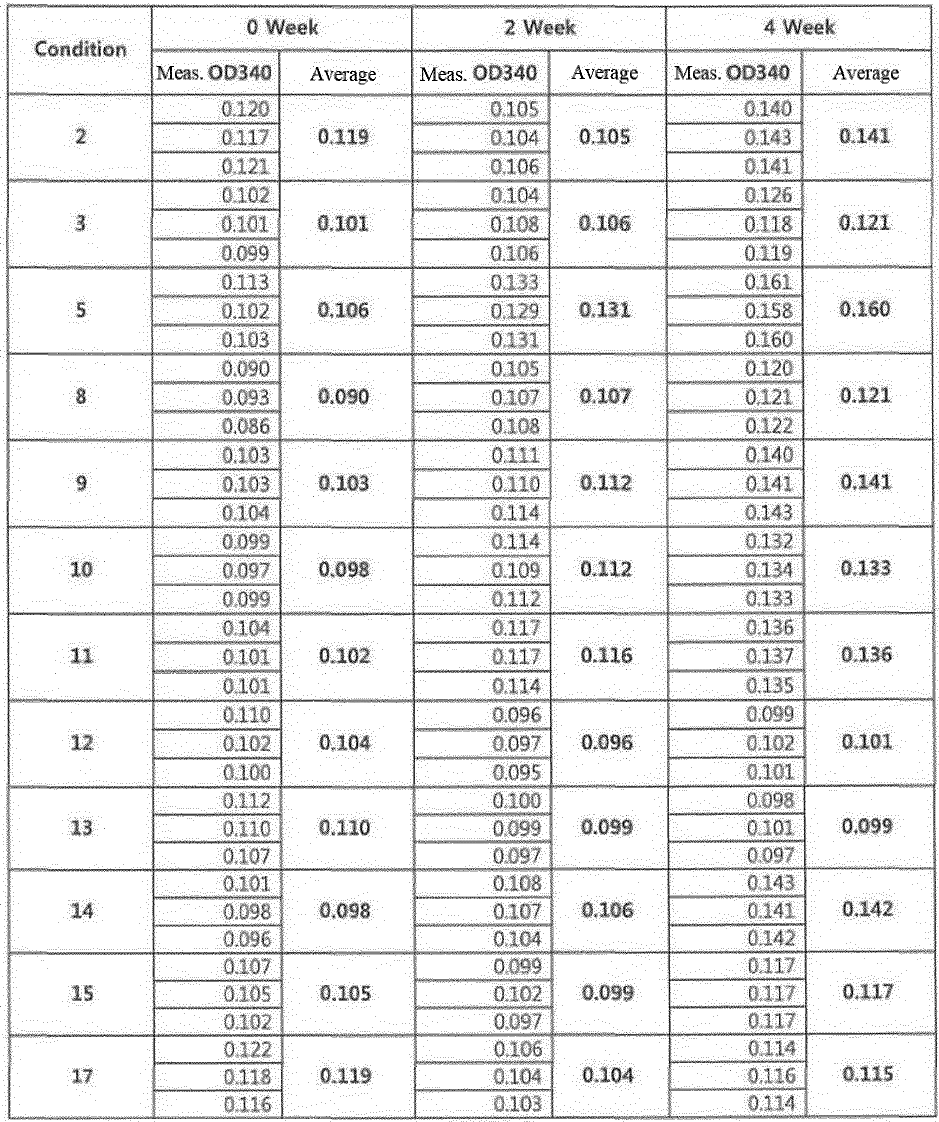

### Example 2.2.2. Results of aggregate and fragment analysis

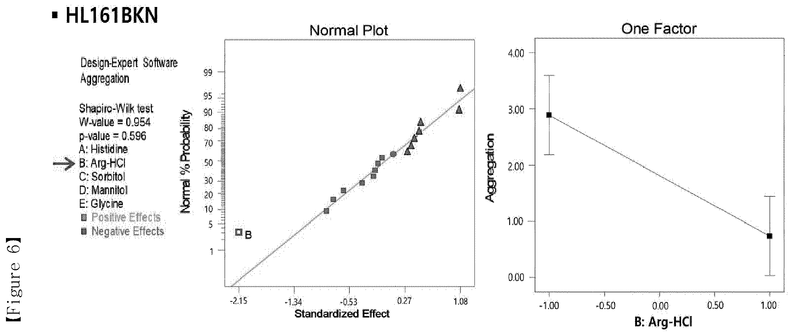

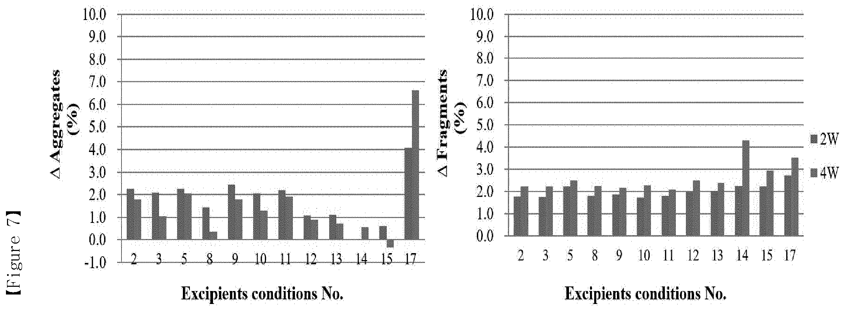

SEC-HPLC was used to compare and evaluate the amount of aggregates and fragments increased for each excipient combination condition (Table 18 and Figure 7). In addition, SEC-HPLC data for a total of 17 conditions, including the excipient only condition test of Example 1.1, was analyzed by ANOVA. As a result, it was confirmed that L-arginine hydrochloride reduced the formation of aggregates at a statistically significant level (p<0.01) (Figure 6), and there was no excipient that reduced the formation of fragments to a statistically significant level.

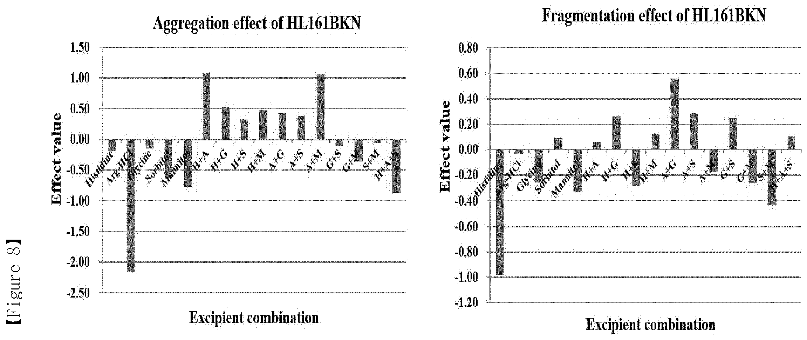

In addition, as a result of comparing the effect values on aggregation and fragmentation, it was confirmed that the formation of aggregates and fragments of HL161BKN was reduced when L-histidine, D-mannitol, and D-sorbitol were used as excipients. However, there was no synergistic effect between the excipients. D-mannitol and D-sorbitol are isomers, and D-mannitol, which is frequently used among them, was selected (Figure 8).

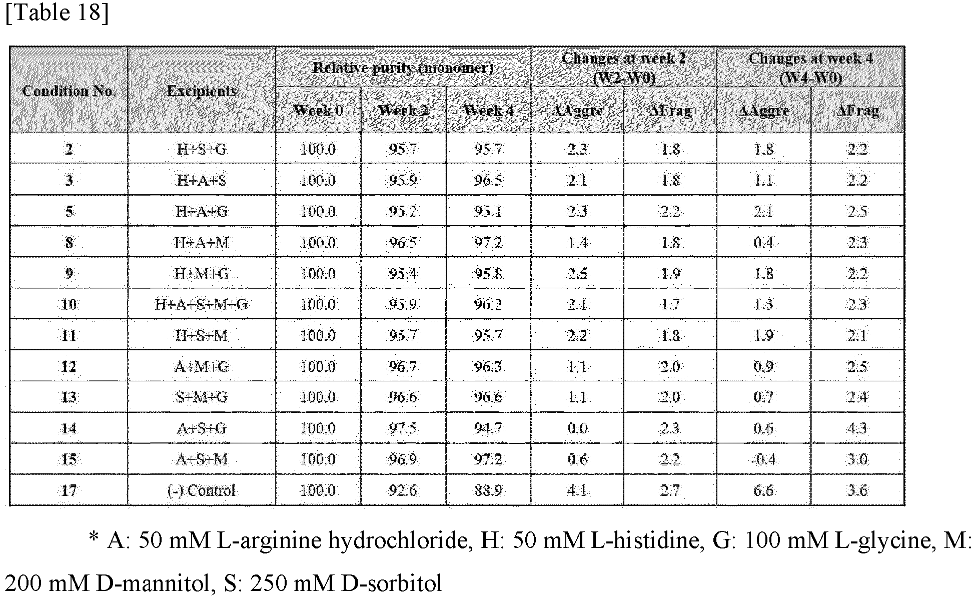

### Example 2.2.3. Results of charge variant analysis

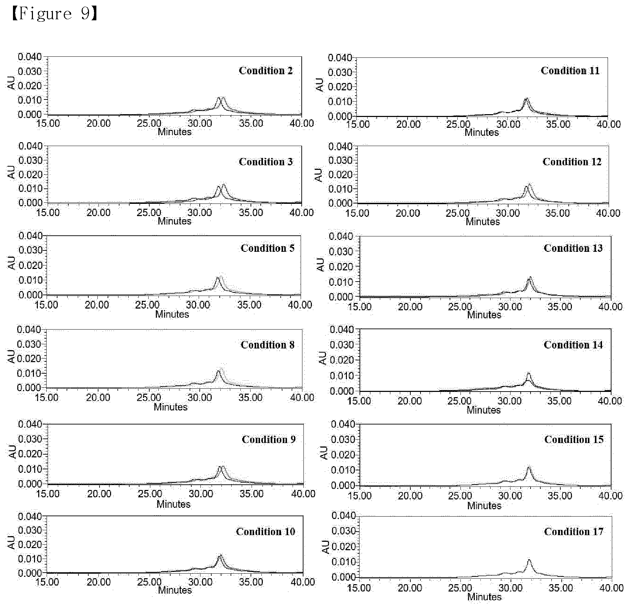

The pattern of changes in charge variants of HL161BKN according to the excipient combination condition was confirmed using CEX-HPLC. As a result, no distinct changes in charge variants (basic and acidic variants) could be observed (Figure 9).

### Example 2.2.4. Results of viscosity and osmolality analysis

As a result of measuring the viscosity for each excipient combination condition, it was confirmed that the viscosity was reduced when L-arginine hydrochloride was added. The osmolality was increased as the number and concentration of added excipients were increased. Specifically, it was confirmed that the addition of 50 mM L-arginine hydrochloride, 50 mM L-histidine, and 100 mM L-glycine increased the osmolality of about 100 mOsmol/kg, respectively; the addition of 200 mM D-mannitol increased the osmolality of about 200 mOsmol/kg; and the addition of 250 mM D-sorbitol increased the osmolality of about 250 mOsmol/kg (Table 19).

In general, the osmolality of subcutaneous injections is similar to the osmolality of the body (about 290 mOsmol/kg) and is regulated in the range of about 250 to 500 mOsmol/kg (PCT/EP2009/066675). Therefore, it was determined that the osmolality of the formulation buffer should be regulated in the range of about 220 to 450 mOsmol/kg in consideration of the HL161BKN concentration. Thus, it was decided to proceed with the subsequent test for excipient concentration screening in consideration of the osmolality range.

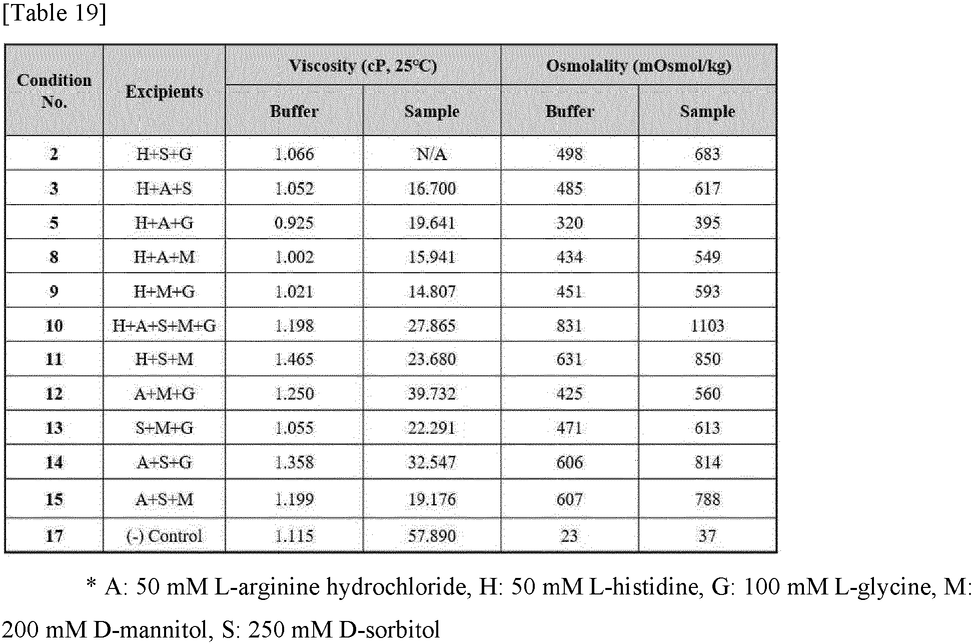

### Example 2.3. Results of additional excipient test

An additional excipient test was performed. As a result, 50 mM histidine (pH 6.0) was used as a basal buffer, and L-methionine, which had the effect of inhibiting the formation of aggregates, and 0.02% PSB20, which inhibited the formation of aggregates under the agitation stress conditions, were selected as additional excipients.

### Example 2.3.1. Results of L-methionine and histidine basal buffer test

### 1) Results of concentration (A280) and turbidity (A340) analysis

The HL161BKN concentration was increased for 4 weeks, which was assumed to be due to buffer evaporation under accelerated conditions of 40°C (Table 20). The turbidity was not increased or showed a slight increase to 0.1 or less (Table 21).

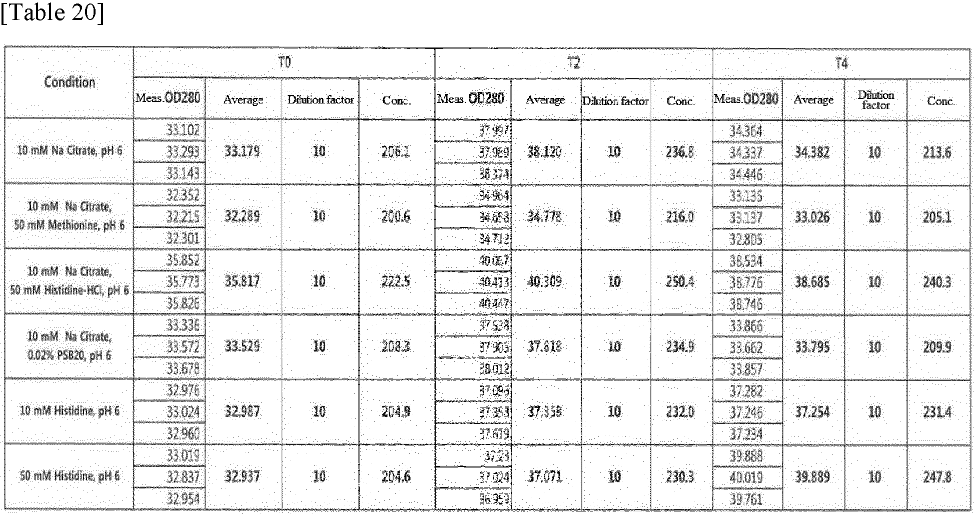

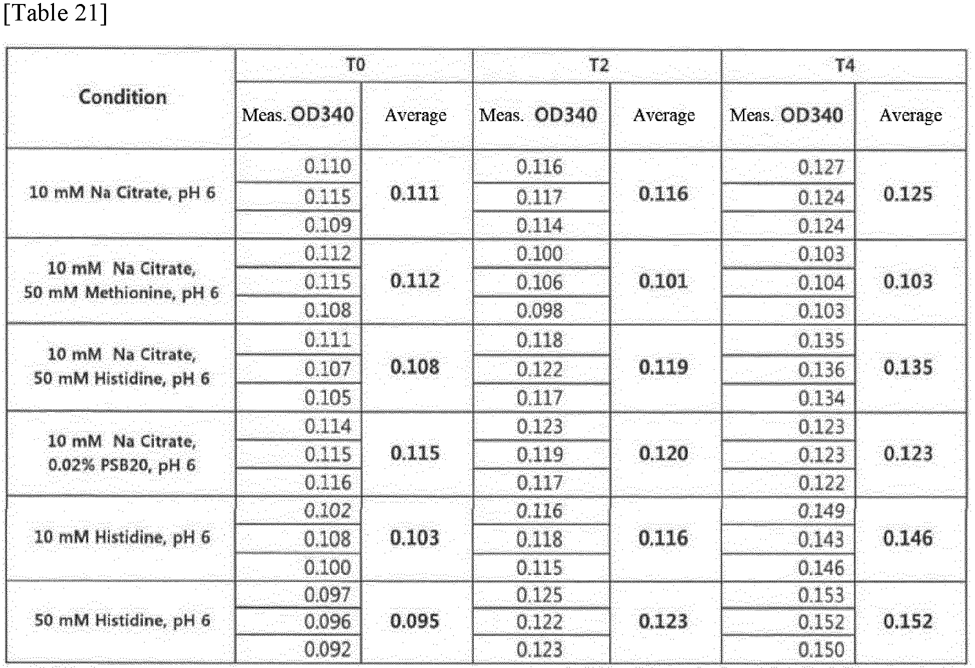

### 2) Results of aggregate and fragment analysis

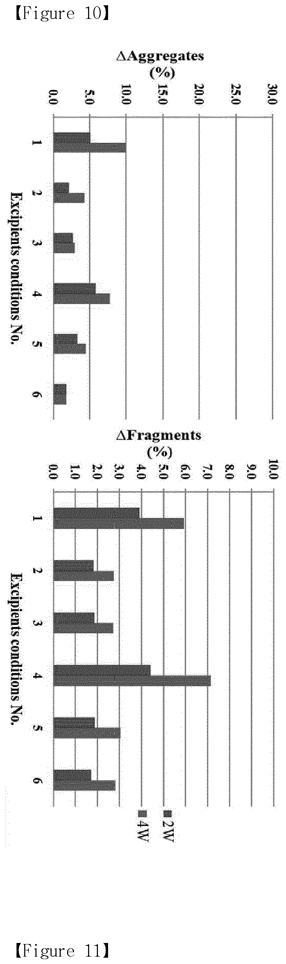

Compared with the basal buffer condition, L-methionine exhibited the excellent effect of inhibiting the formation of aggregates. In addition, there was no increase in the formation of aggregates by 0.02% PSB20. In addition, the results of the condition in which L-histidine was added as an excipient was similar to that of the condition in which it was used as a basal buffer, and the formation of aggregates was reduced in the 50 mM histidine condition compared to that in the 10 mM histidine condition (Table 22 and Figure 10).

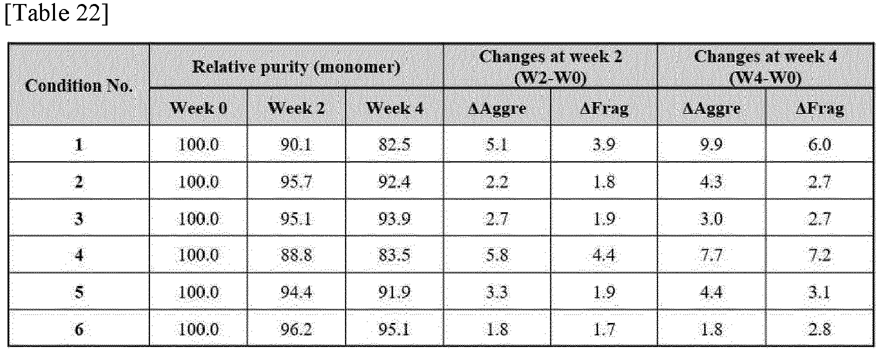

### 3) Results of charge variant analysis

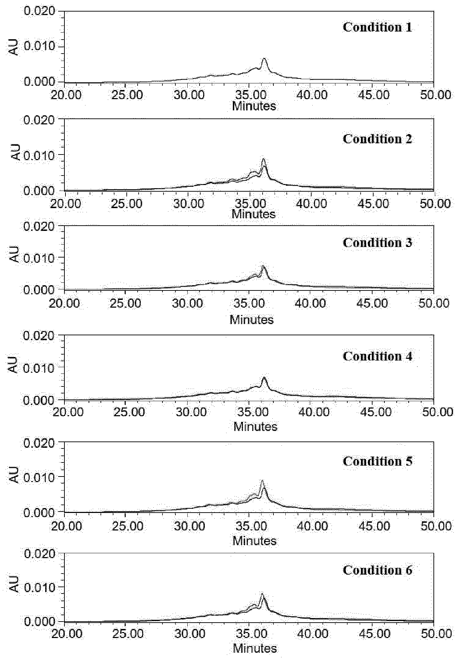

As a result of CEX-HPLC analysis, no distinct changes in charge variants (basic and acidic variants) could be observed (Figure 11).

### Example 2.3.2. Results of polysorbate 20 test

### 1) Results of concentration (A280) and turbidity (A340) analysis

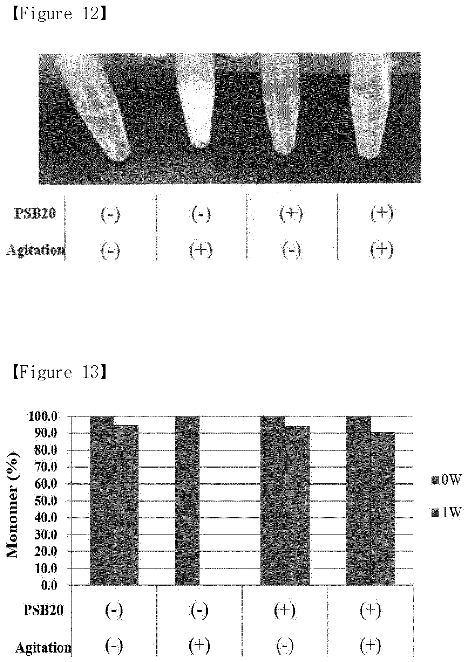

As a result of the agitation test at room temperature, there was little change in concentration. The sample agitated without PSB20 turned white visually and the turbidity could not be measured (Figure 12). On the other hand, the sample with PSB20 was visually slightly cloudy under agitation condition, and the turbidity was increased by about 0.227 (Table 23).

Through this, it was confirmed that PSB20 has an effect of protecting high concentration HL161BKN against the stress caused by agitation.

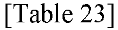

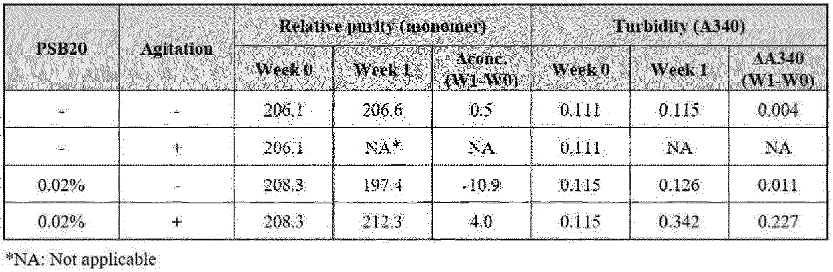

### 2) Results of aggregate and fragment analysis

The formation of aggregates was increased by agitation stress, and it was confirmed that PSB20 had the effect of inhibiting the formation of aggregates. On the other hand, there was no increase in fragments due to agitation stress (Table 24 and Figure 13).

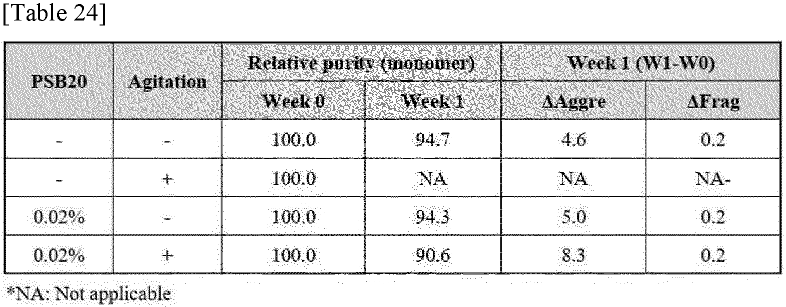

### Example 2.3.3. Results of screening of excipient for viscosity reduction

### 1) Results of concentration (A280) and viscosity analysis

The HL161BKN concentration was increased for 4 weeks (Table 25), which was assumed to be due to buffer evaporation under accelerated conditions of 40°C. When L-histidine hydrochloride, L-arginine hydrochloride, and L-glycine were added, the viscosity was reduced compared to Condition 1, but the effect therby was at an insignificant. On the other hand, in the case of L-lysine hydrochloride, NaCl, Na₂SO₄, and NH₄Cl, the viscosity was rather greatly increased (Table 26).

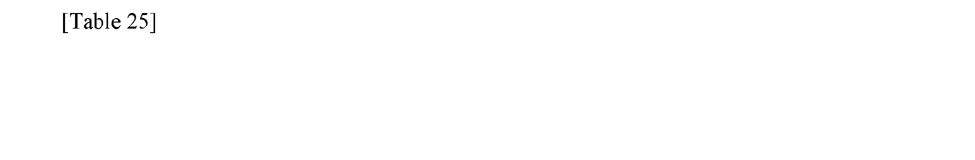

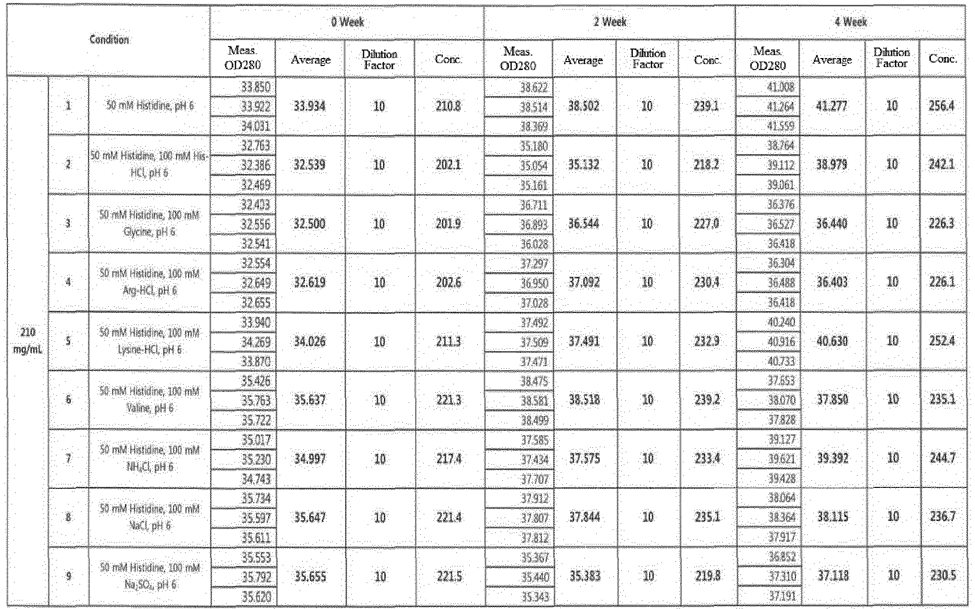

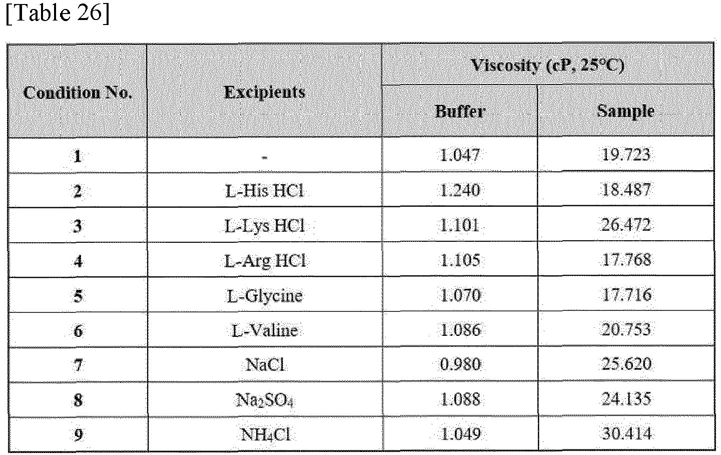

### 2) Results of aggregate and fragment analysis

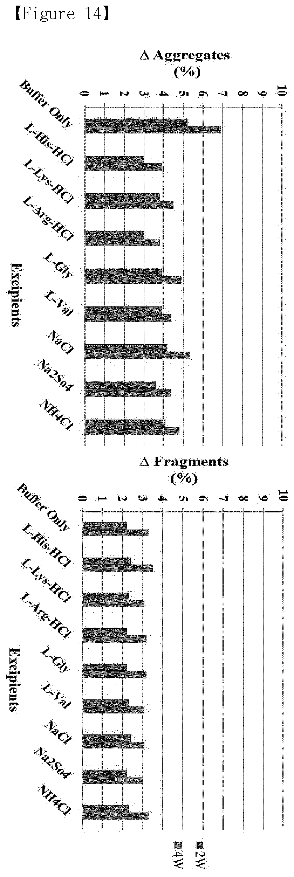

The aggregates and fragments were analyzed by SEC-HPLC analysis. As a result, when L-histidine hydrochloride and L-arginine hydrochloride were added, relatively few aggregates were formed compared to Condition 1, and there was no excipient to reduce the formation of fragments (Table 27 and Figure 14).

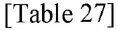

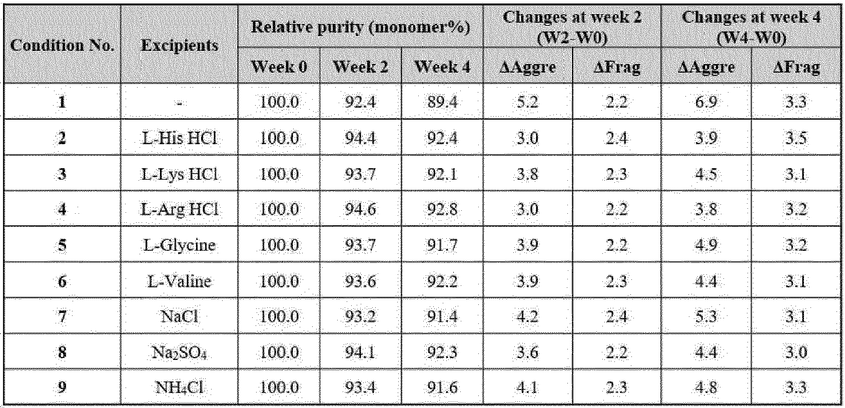

### Example 2.4. Results of excipient concentration screening

### Example 2.4.1. Results of first excipient concentration screening

In order to screen the concentration of the histidine basal buffer (pH 6.0) selected in Example 1.3 above and three excipients (L-methionine, L-arginine hydrochloride, and D-mannitol) in 0.02% PSB20, a test was performed under a total of 9 conditions (Table 28). As a result, L-arginine hydrochloride and PSB20 were selected as excipients in addition to the histidine basal buffer.

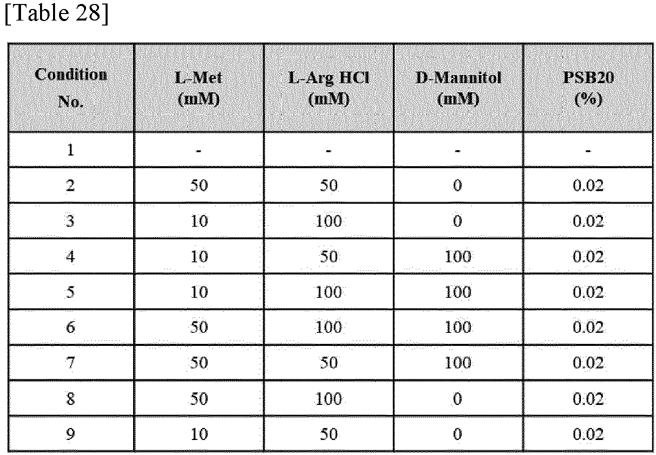

### 1) Results of concentration (A280) and turbidity (A340) analysis

The HL161BKN concentration was increased for 4 weeks by about 15%, which was assumed to be due to buffer evaporation under accelerated conditions of 40°C (Table 29). The turbidity in each condition showed only an insignificant increase of about 0.048 on average (Table 30).

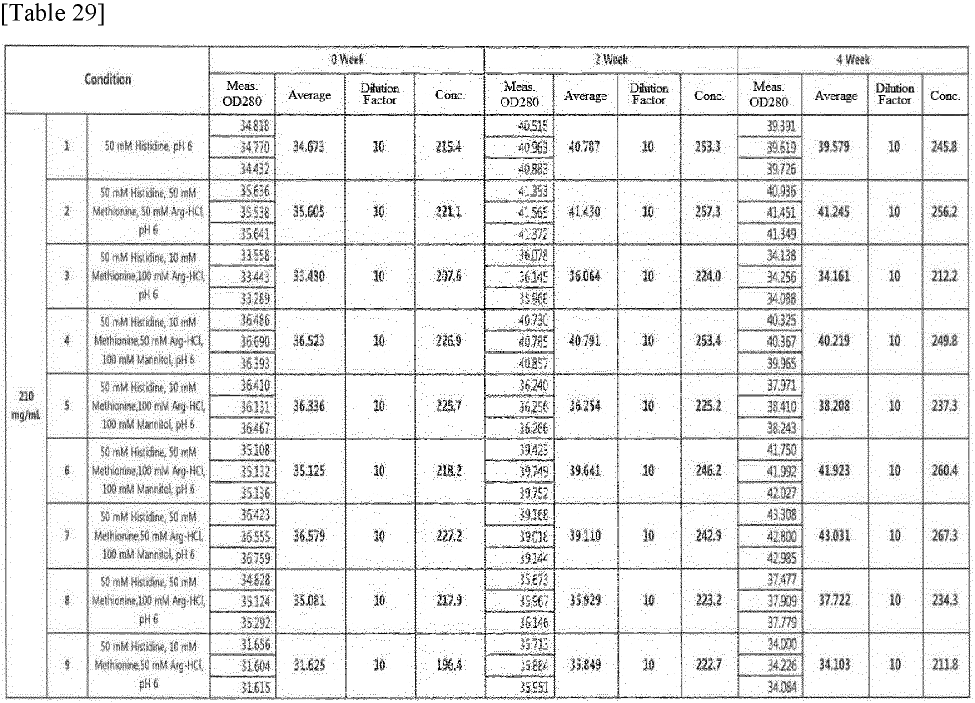

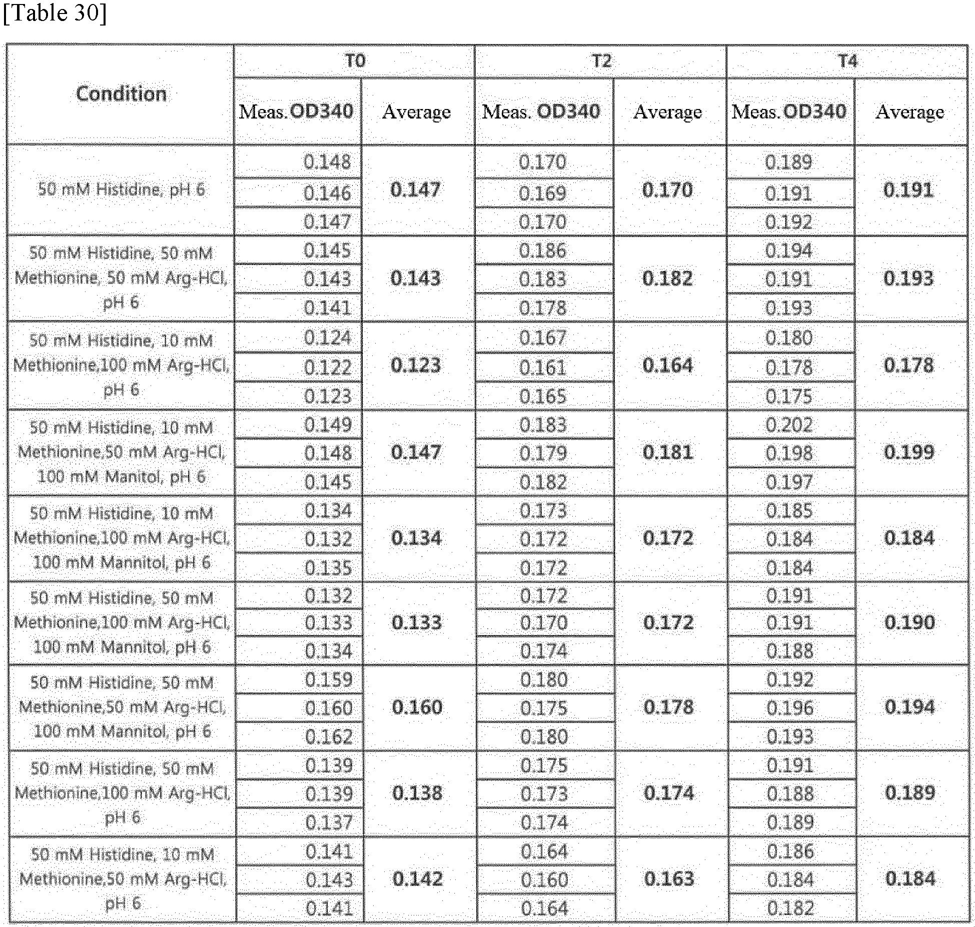

### 2) Results of aggregate and fragment analysis

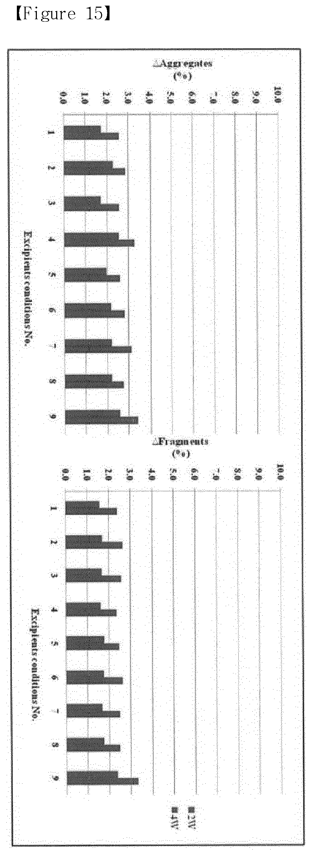

As a result of SEC-HPLC analysis, the amount of aggregates and fragments increased according to each excipient concentration condition did not show a significant difference. However, the stability in the basal buffer condition (Condition 1) of 50 mM histidine was high, and the formation of aggregates was reduced when 100 mM L-arginine hydrochloride was added rather than 50 mM. In addition, it was confirmed that the amount of aggregates and fragments increased according to the concentration of L-methionine and the presence or absence of D-mannitol added had little difference or a slight increase from the basal buffer condition (Table 31 and Figure 15).

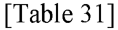

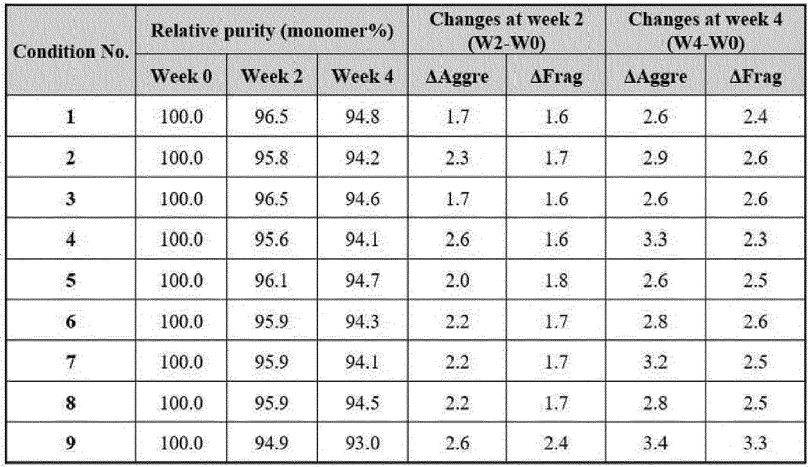

### 3) Results of charge variant analysis

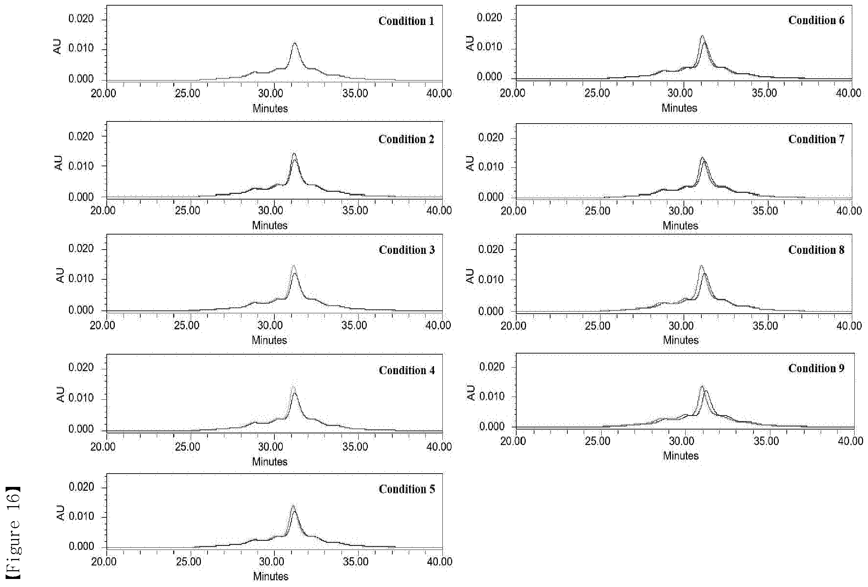

As a result of CEX-HPLC analysis, no distinct changes in charge variants were observed according to each excipient condition (Figure 16).

### 4) Results of viscosity and osmolality analysis

As a result of measuring the viscosity and osmolality according to each excipient concentration condition, it was confirmed that the effect of reducing the viscosity was high in the condition in which L-arginine hydrochloride was added at a concentration of 100 mM rather than 50 mM, and most of them had 112 to 588 mOsmol/kg to satisfy the osmolality criteria (Table 32).

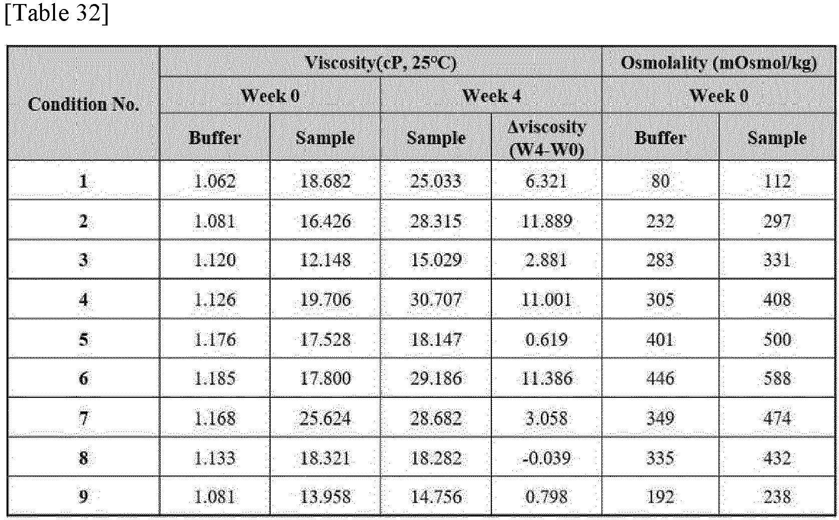

### Example 2.4.2. Second excipient concentration screening

Among the histidine basal buffer selected in the first excipient concentration screening of Example 1.4.1 and two excipients, in order to select the optimal concentration of L-arginine hydrochloride, 2 batch samples of HL161BKN (HL161BKN B018 and HL161BKN B021) were evaluated for the stability through the concentration, turbidity, purity (aggregates and fragments), viscosity, osmolality test, and suitability for subcutaneous injection (SC) administration in four conditions. As a result, 100 mM L-histidine, 100 mM L-arginine hydrochloride, 0.02% PSB20, pH 6.0 was determined as the final formulation of HL161BKN (Table 33).

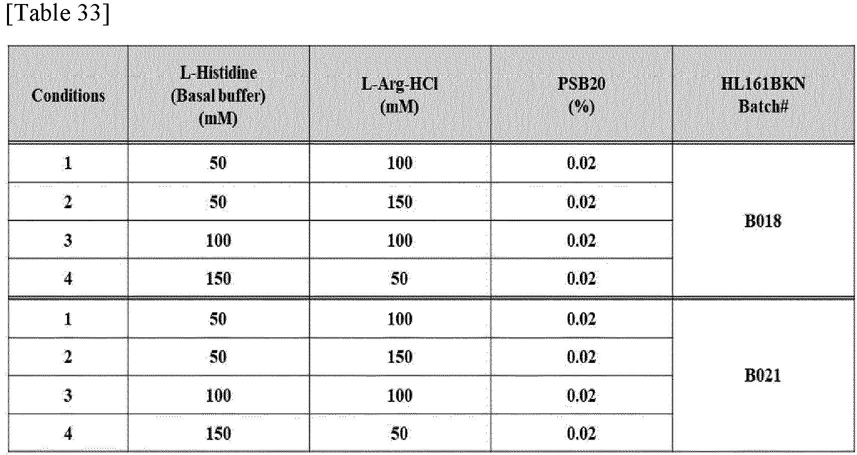

### 1) Concentration (A280) and turbidity (A340) analysis

There was little change in the HL161BKN concentration for 4 weeks, which was expected to be an effect of preventing evaporation of the buffer by sealing the 1.5 mL microcentrifuge with parafilm and then storing it in a thermo-hygrostat (Table 34). In addition, the turbidity under all conditions showed an insignificant increase of 0.102 or less (Table 35).

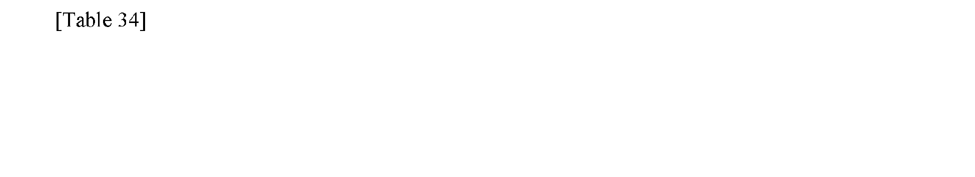

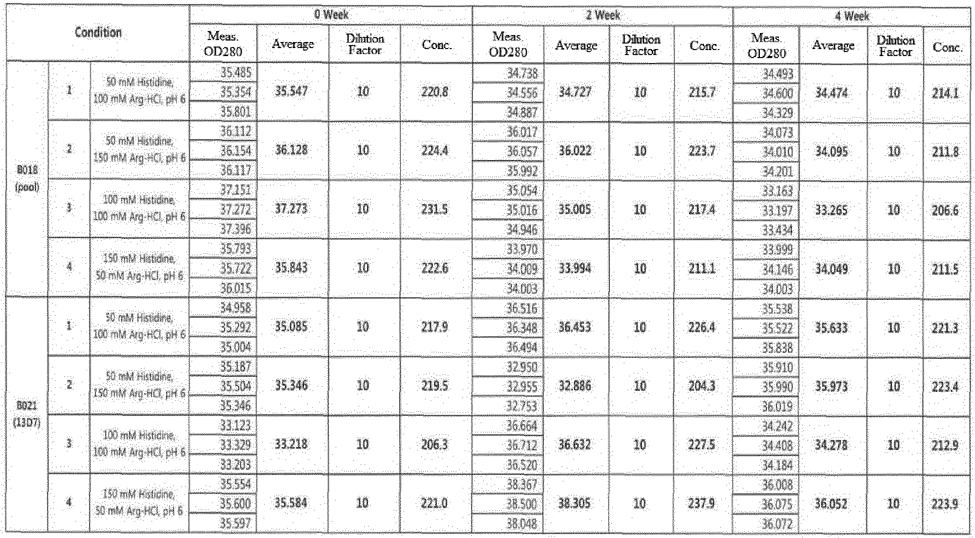

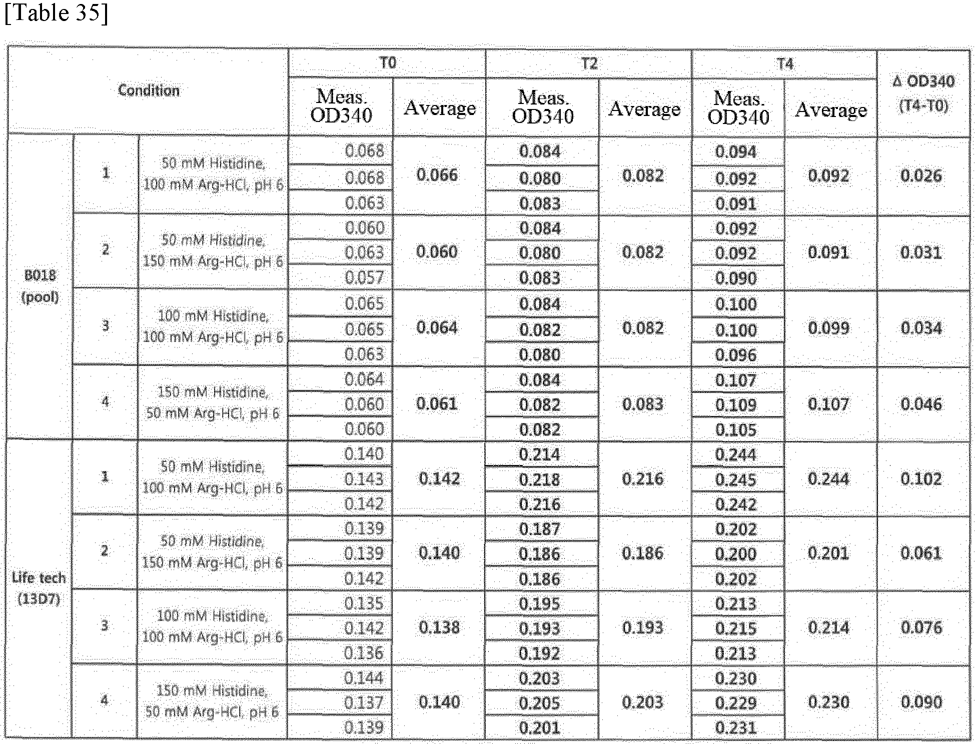

### 2) Aggregate and fragment analysis

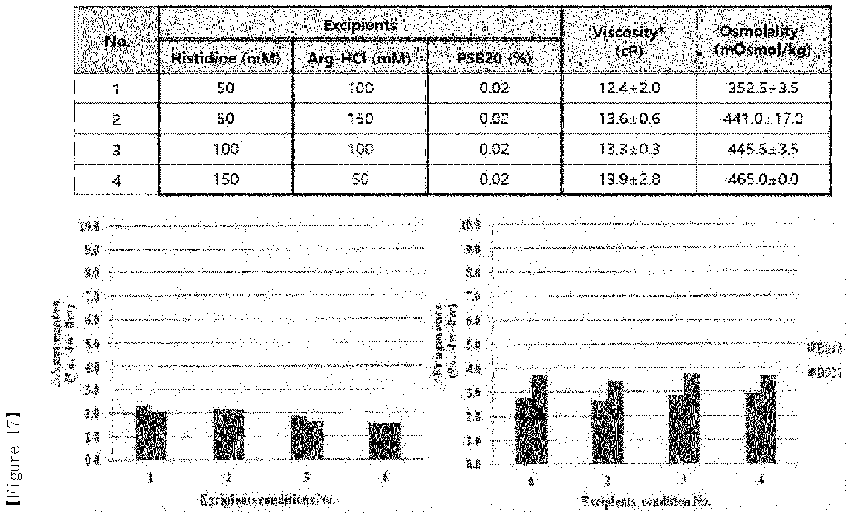

Through SEC-HPLC analysis, the amount of aggregates and fragments increased according to each excipient condition did not show a significant difference, but it was confirmed that the formation of aggregates was the least in Condition 3 (100 mM L-Histidine, 100 mM L-Arginine HCl, 0.02% PSB20, pH 6.0) and Condition 4 (50 mM L-Histidine, 100 mM L-Arginine HCl, 0.02% PSB20, pH 6.0) (Table 36 and Figure 17).

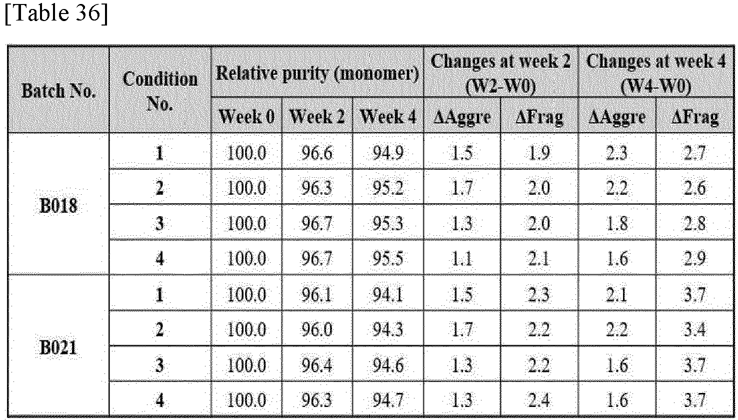

### 3) Charge variant analysis

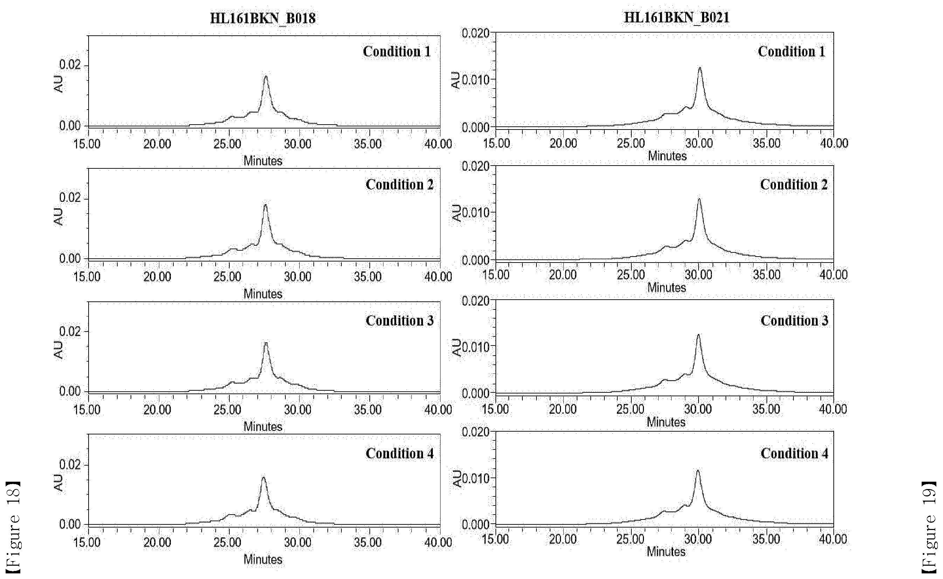

As a result of CEX-HPLC analysis, no distinct changes in charge variants (basic and acidic variants) was observed according to each excipient condition (Figure 18).

### 4) Viscosity and osmolality analysis

As a result of measuring the viscosity and osmolality according to each excipient concentration condition, the viscosity was regulated to 11 to 16 cP, i.e., below the 20 cp of limit, which is the viscosity limit of the subcutaneous administration product, and the osmolality was 350 to 465 mOsmol/kg to satisfy the criteria of 250 to 500 mOsmol/kg (Table 37).

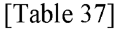

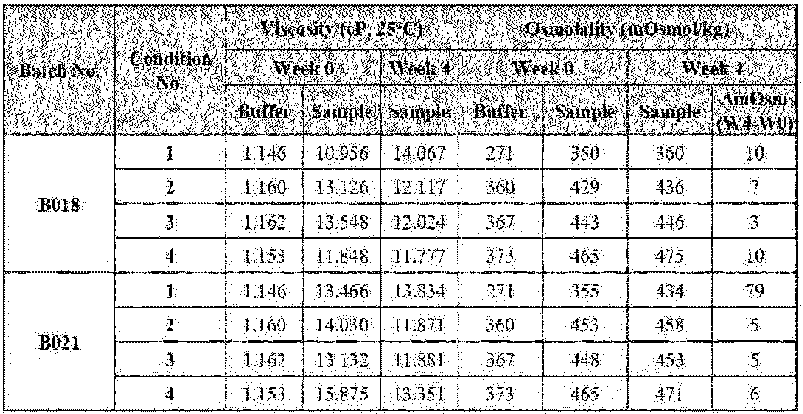

### 5) Analysis of insoluble sub-visible particles

Analysis of insoluble sub-visible particles for each excipient condition sample of HL161BKN using micro flow imaging (MFI) was conducted at New Drug Development Support Center of Osong Hi-tech Medical Industry Promotion Foundation.

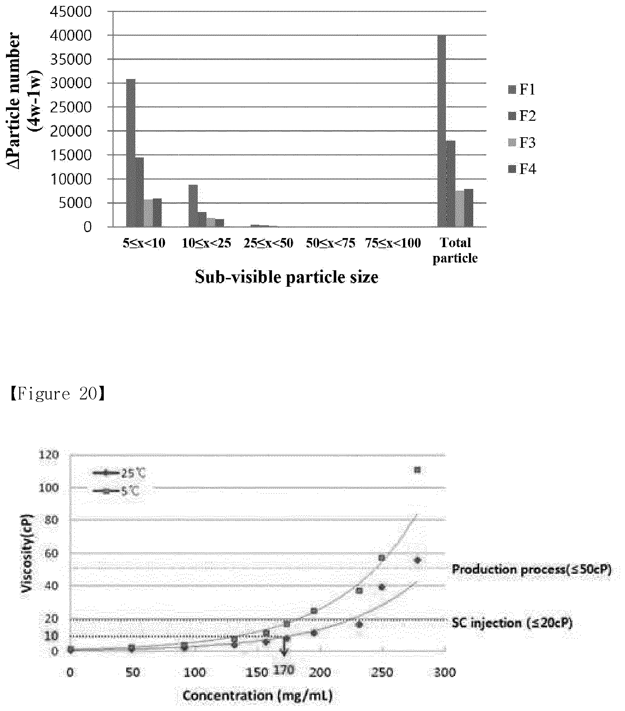

Samples of each condition were diluted to 10 mg/mL to compare the increase in the number of sub-visible particles in the range of 5 µm to 100 µm. As a result, the increase in the number of sub-visible particles was the least in Condition 3 (Table 38 and Figure 19).

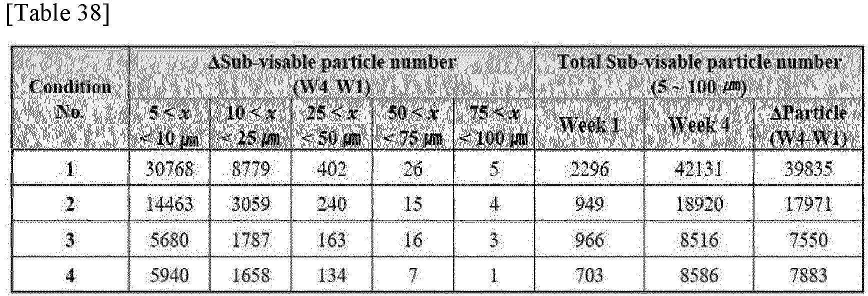

### Example 3. Stability test

A 36-month long-term storage and accelerated stability test under the final selected formulation conditions for HL161BKN was performed by Catalent (USA). In order to evaluate the stability, the drug product (DP) was stored using a borosilicate glass vial and a teflon-coated rubber stopper. As a result of confirming the stability under the selected formulation conditions, the stability of DP was confirmed for 36 months, thereby securing the possibility of development as an injection (Table 39 and Table 40).

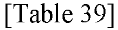

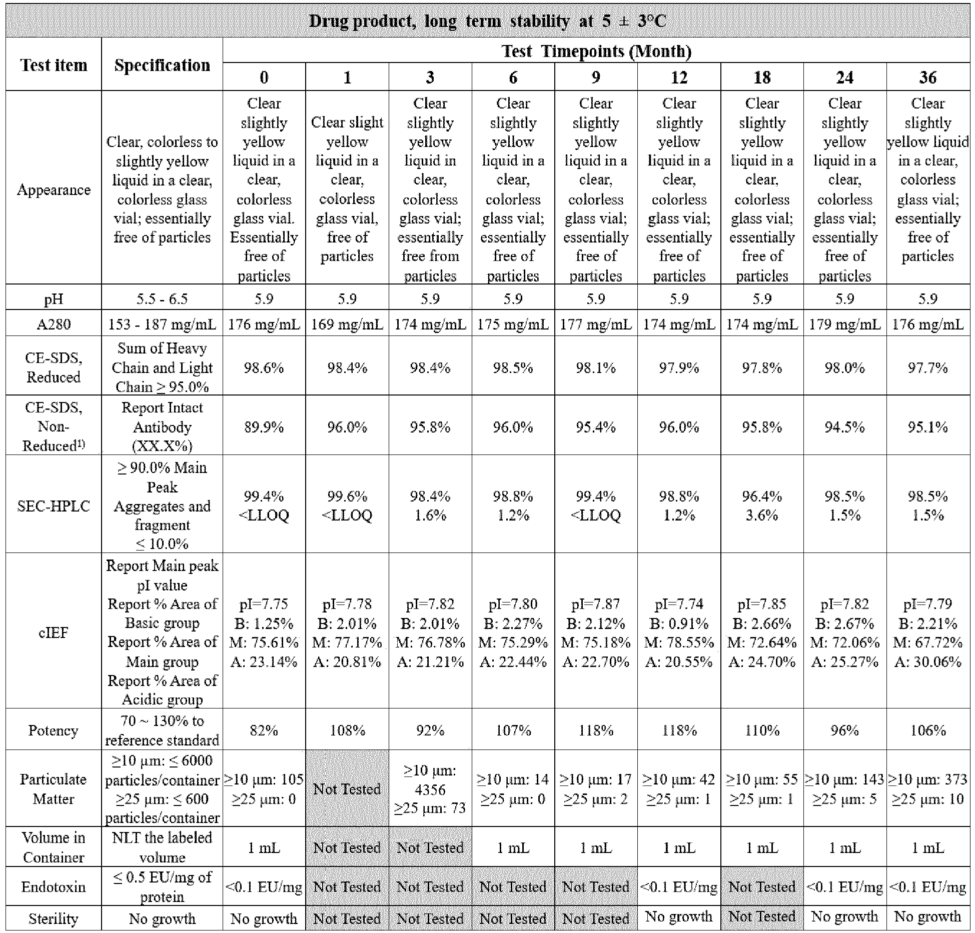

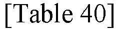

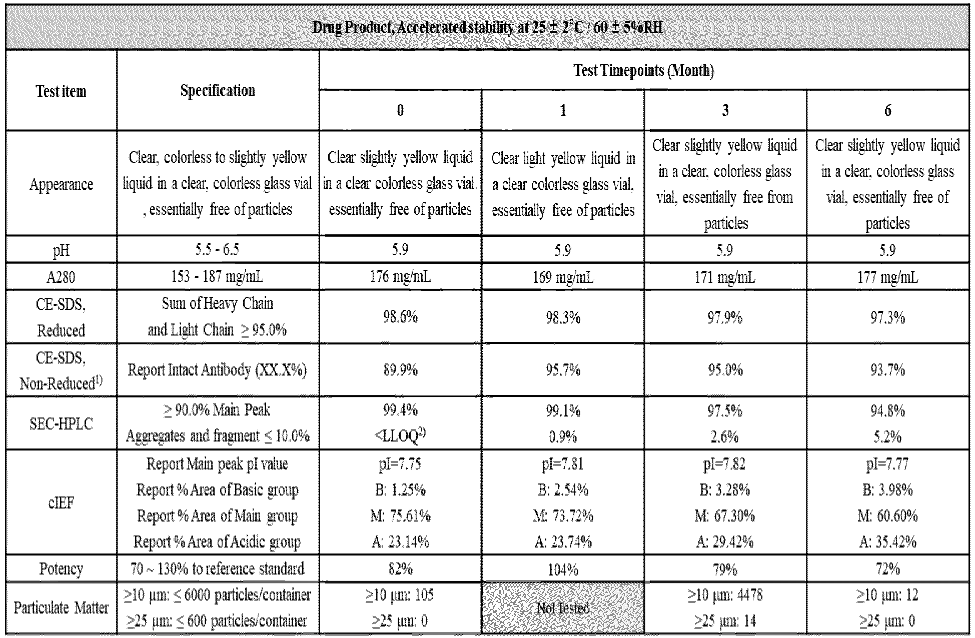

### Example 4. Selection of final development candidate antibody formulation

Since HL161BKN exhibits a tendency to be very stable at a high concentration in the formulation, it was intended to confirm the possibility of development in the form of an injection for subcutaneous administration. When developing an injection, it is known that a viscosity of 20 cP or less is suitable for subcutaneous administration in order to reduce pain and side effects at the site of administration. Accordingly, HL161BKN was concentrated under 9-step concentration conditions, and the viscosity was measured under 5°C and 25°C conditions. As a result of confirming the viscosity of the HL161BKN sample having a high concentration of 170 mg/mL, it was confirmed that it had a viscosity of 10 cP at 25°C, and thus subcutaneous administration was possible (Figure 20).

It was confirmed that HL161BKN had very stable properties even at a high concentration of 200 mg/mL or more in the above formulation study. As a result, it is expected that it will be possible to develop a self-administered SC injection product in the future. Considering that all products of other competing companies are infusion type products, it is possible to be differentiated by increasing patient convenience.

In addition, since it was confirmed that a high concentration of HL161BKN was stable in the formulation, a low concentration of HL161BKN was also expected to be stable in the formulation. Therefore, the formulation can be applied to HL161BKN at various concentrations.

## Claims

1. A pharmaceutical formulation having a pH of 4.0 to 8.0, comprising
(a) an anti-FcRn antibody or a fragment thereof,
(b) at least one additive selected from mannitol, sorbitol, arginine, histidine, glycine and salts thereof,
(c) a buffer system selected from citrate or histidine, and
(d) a surfactant.

2. The pharmaceutical formulation according to claim 1, wherein the additive is arginine or a salt thereof, and the buffer system is histidine.

3. The pharmaceutical formulation according to claim 1, wherein the surfactant is polysorbate.

4. The pharmaceutical formulation according to claim 1, further comprising methionine.

5. The pharmaceutical formulation according to claim 1, wherein the pharmaceutical formulation is in the form of an injection.

6. The pharmaceutical formulation according to claim 1, wherein the pharmaceutical formulation has a viscosity of 20 cP or less.

7. The pharmaceutical formulation according to claim 1, wherein the pharmaceutical formulation has an osmolality of 250 mOs/kg to 500 mOs/kg.

8. The pharmaceutical formulation according to claim 1, wherein the concentration of the anti-FcRn antibody or fragment thereof is 50 mg/mL to 250 mg/mL.

9. The pharmaceutical formulation according to claim 8, wherein the concentration of the anti-FcRn antibody or fragment thereof is 80 mg/mL to 250 mg/mL.

10. The pharmaceutical formulation according to claim 1, wherein the pH of the pharmaceutical formulation is 4.0 to 7.0.

11. The pharmaceutical formulation according to claim 1, wherein the anti-FcRn antibody comprises
a heavy chain variable region comprising H-CDR1 having the amino acid of SEQ ID NO: 5, H-CDR2 having the amino acid of SEQ ID NO: 6, and H-CDR3 having the amino acid of SEQ ID NO: 7; and
a light chain variable region comprising L-CDR1 having the amino acid of SEQ ID NO: 8, L-CDR2 having the amino acid of SEQ ID NO: 9, and L-CDR3 having the amino acid of SEQ ID NO: 10.

12. The pharmaceutical formulation according to claim 1, wherein the pharmaceutical formulation is administered subcutaneously.

13. The pharmaceutical formulation according to claim 1, wherein the pharmaceutical formulation comprises
(a) 50 mg/mL to 250 mg/mL of an anti-FcRn antibody,
(b) 50 to 250 mM L-arginine or a hydrochloride salt thereof,
(c) 50 to 250 mM L-histidine buffer, and
(d) 0.01 to 0.05% polysorbate 20, and
the pharmaceutical formulation has a pH of 4.0 to 7.0.

14. The pharmaceutical formulation according to claim 1, wherein the pharmaceutical formulation has increased stability, wherein the amount of aggregates and fragments of HL161BKN is 10% or less when stored for 6 months under an accelerated condition (25°C, a relative humidity of 60%).

15. The pharmaceutical formulation according to claim 1, wherein the pharmaceutical formulation has increased stability, wherein the amount of aggregates and fragments of HL161BKN is 10% or less when stored for 36 months under a long-term storage condition (5°C).

16. The pharmaceutical formulation according to claim 1, wherein the pharmaceutical formulation has increased stability, wherein the amount of aggregates and fragments of HL161BKN is 5.0% or less when stored for 36 months under a long-term storage condition (5°C).

17. The pharmaceutical formulation according to any one of claims 1 to 16, wherein the pharmaceutical formulation is for the treatment of an autoimmune disease selected from the group consisting of myasthenia gravis, thyroid eye disease, warm autoimmune hemolytic anemia, neuromyelitis optica, immune thrombocytopenic purpura, pemphigus vulgaris, chronic inflammatory demyelinating polyneuropathy, lupus nephritis, and membranou
